(19) Europäisches Patentamt
European Patent Office
Office européen des brevets

(11) Publication number: **0 163 237**
**A2**

(12) # EUROPEAN PATENT APPLICATION

(21) Application number: 85106201.8

(22) Date of filing: 21.05.85

(51) Int. Cl.[4]: **C 07 K 5/06**
C 07 K 5/08, C 07 K 5/10
C 07 K 7/00, A 61 K 37/02

(30) Priority: 29.05.84 US 614881

(43) Date of publication of application:
04.12.85 Bulletin 85/49

(84) Designated Contracting States:
CH DE FR GB IT LI NL

(71) Applicant: MERCK & CO. INC.
126, East Lincoln Avenue P.O. Box 2000
Rahway New Jersey 07065(US)

(72) Inventor: Boger, Joshua S.
719 New Gulph Road
Bryn Mawr Pennsylvania 19010(US)

(72) Inventor: Bock, Mark G.
1603 Leon Drive
Hatfield Pennsylvania 19440(US)

(72) Inventor: Freidinger, Roger
2185 Rebecca Drive
Hatfield Pennsylvania 19440(US)

(72) Inventor: Veber, Daniel F.
290 Batleson Road
Ambler Pennsylvania 19002(US)

(74) Representative: Abitz, Walter, Dr.-Ing. et al,
Abitz, Morf, Gritschneder, Freiherr von Wittgenstein
Postfach 86 01 09
D-8000 München 86(DE)

(54) **Di- and tri-peptidal renin inhibitors.**

(57) Renin inhibitory peptides of the formula

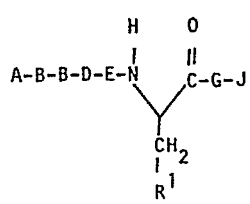

(1.)

and analogs thereof inhibit renin and are useful for treating various forms of renin-associated hypertension and hyperaldosteronism.

EP 0 163 237 A2

TITLE OF THE INVENTION

DI- AND TRI-PEPTIDAL RENIN INHIBITORS

BACKGROUND OF THE INVENTION

1. Field of The Invention

The present invention is concerned with novel peptides which inhibit renin.

The present invention is also concerned with pharmaceutical compositions containing the novel peptides of the present invention as active ingredients, with methods of treating renin-associated hypertension and hyperaldosteronism, with diagnostic methods which utilize the novel peptides of the present invention, and with methods of preparing the novel peptides of the present invention.

Renin is a proteolytic enzyme of molecular weight about 40,000, produced and secreted by the kidney. It is secreted by the juxtaglomerular cells and acts on the plasma substrate, angiotensinogen, to split off the decapeptide angiotensin I, which is

converted to the potent pressor agent angiotensin II. Thus, the renin-angiotensin system plays an important role in normal cardiovascular homeostasis and in some forms of hypertension.

In the past, attempts to modulate or manipulate the renin-angiotensin system have met with success in the use of inhibitors of angiotensin I converting enzyme. In view of this success, it seems reasonable to conclude that a specific inhibitor of the limiting enzymatic step that ultimately regulates angiotensin II production, the action of renin on its substrate, would be at least equally successful. Thus, an effective inhibitor of renin has been long sought as both a therapeutic agent and as an investigative tool.

2. Brief Description of the Prior Art

There has been substantial interest in the synthesis of useful renin inhibitors for many decades; and the following table lists the major classes of renin inhibitors that have been studied, as well as their inhibition constants ($K_i$):

| Class | $K_i$ (M) |
|---|---|
| Renin antibody | probably $10^{-6}$ |
| Pepstatin | $10^{-6}$ - $10^{-7}$ |
| Phospholipids | $10^{-3}$ |
| Substrate analogs | |
| Tetrapeptides | $10^{-3}$ |
| Octa- to tridecapeptides | $10^{-5}$ - $10^{-6}$ |

Umezawa et al., in J. Antibiot. (Tokyo) 23: 259-262, 1970, reported the isolation of a peptide from actinomyces that was an inhibitor of aspartyl proteases such as pepsin, cathepsin D, and renin.

This peptide, known as pepstatin, was found by Gross et al., Science 175:656, 1971, to reduce blood pressure in vivo after the injection of hog renin into nephrectomized rats. However, pepstatin has not found wide application as an experimental agent because of its limited solubility and its inhibition of a variety of other acid proteases in addition to renin. The structure of pepstatin is shown below:

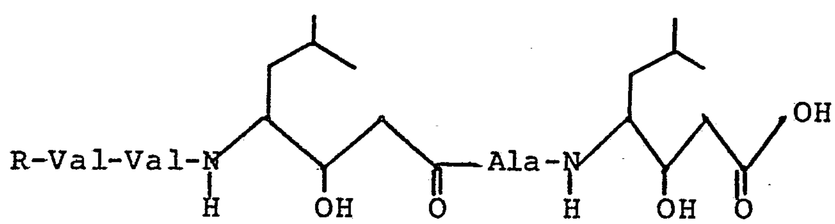

To date, many efforts have been made to prepare a specific renin inhibitor based on substrate analogy. Since the human renin substrate has only recently been elucidated (Tewksbury et al., Circulation 59, 60, Supp. II: 132, Oct. 1979), heretofore substrate analogy has been based on the known pig renin substrate. While the human and pig renin substrates are not the same, the substrate analogy based on pig renin has always been considered acceptable in the art as predictive of human renin inhibitory activity because of the closely related activity of the two renins. Thus, while pig renin does not cleave the human renin substrate, human renin, on the other hand, does cleave the pig renin substrate. See Poulsen et al., Biochim. Biophys. Acta 452:533-537, 1976; and Skeggs, Jr. et al., J. Exp. Med. 106:439-453, 1957. Moreover, the human renin inhibitory activity of the peptides of the present

invention most active in inhibiting pig renin has been confirmed, thus providing further evidence of this accepted correlation between human and pig renin activity.

It has been found, for example, using pig renin substrate analogy, that the octapeptide sequence extending from histidine-6 through tyrosine-13 has kinetic parameters essentially the same as those of the full tetradecapeptide renin substrate. The amino acid sequence of the octapeptide in pig renin substrate is as follows:

6 7 8 9 10 11 12 13
-His-Pro-Phe-His-Leu-Leu-Val-Tyr-

Renin cleaves this substrate between $Leu^{10}$ and $Leu^{11}$.

Kokubu et al., Biochem. Pharmacol. 22: 3217-3223, 1973, synthesized a number of analogs of the tetrapeptide found between residues 10 to 13, but while inhibition could be shown, inhibitory constants were only of the order of $10^{-3}$M.

Analogs of a larger segment of renin substrate were also synthesized: Burton et al., Biochemistry 14: 3892-3898, 1975, and Poulsen et al., Biochemistry 12: 3877-3882, 1973. Two of the major obstacles which had to be overcome to obtain an effective renin inhibitor useful in vivo were lack of solubility and weak binding (large inhibitory constant). Modifications to increase solubility soon established that the inhibitory properties of the peptides are markedly dependent on the hydrophobicity of various amino acid residues, and that increasing

solubility by replacing lipophilic amino acids with hydrophilic isosteric residues becomes counterproductive. Other approaches to increasing solubility have had limited success. Various modifications designed to increase binding to renin have also been made, but here too, with only limited success. For a more detailed description of past efforts to prepare an effective inhibitor of renin, see Haber and Burton, Fed. Proc. Fed. Am. Soc. Exp. Biol. 38: 2768-2773, 1979.

More recently, Szelke et al., in work described in European Patent Publication No. 45,665; Nature, 299, 555 (1982); Hypertension, 4, Supp. 2, 59, 1981; British Patent No. 1,587,809; and "Novel Transition-State Analogue Inhibitors of Renin", a presentation at the Eighth American Peptide Symposium, May 22-27, 1983, Tucson, Arizona, have replaced the Leu-Leu site of renin cleavage by isosteric substitution, and obtained compounds with excellent potency.

Powers et al., in Acid Proteases, Structure, Function and Biology, Plenum Press, 1977, 141-157 have suggested that in pepstatin, statine occupies the space of the two amino acids on either side of the cleavage site of a pepsin substrate, and Tang et al., in Trends in Biochem. Sci., 1:205-208 (1976) and J. Biol. Chem., 251:7088-94, 1976, have proposed that the statine residue of pepstatin resembles the transition state for pepsin hydrolysis of peptide bonds. However, the applicability of these concepts to renin inhibitors is not taught in any of these references, and would be speculative due to the known high degree of specificity of the renin enzyme.

Kokubu et al., Biochem. Biophys. Res. Comm. 118:929-933, 1984; and Fehrentz et al., FEBS Letters 167: 273-276, 1984, have prepared a renin inhibitor in which a C-terminal aldehyde is used to mimic $Leu^{10}$ of the substrate. However, there is no suggestion of the renin inhibitors of the present invention in which statine and other moieties replace $Leu^{10}$-$Leu^{11}$ of the substrate.

Veber and Rich, in U.S. Patent No. 4,384,994 and published European Patent Application No. 0,077,029; Evans and Rittle, in U.S. Patent No. 4,397,786; Veber and Boger, in published European Patent Application No. 0,077,028; Boger et al, Nature, 303:81-84 (1983); have all described renin inhibitory peptides containing statine; and in Nature there is further described renin inhibitors having a shortened C-terminus, with a non-peptide ending after the 11-position. However, none of these references describe or suggest the renin inhibitors of the present invention and the significant increase in renin inhibitory activity obtainable therewith. Moreover, the Nature reference teaches away from renin inhibitors having non-peptide components after the 11-position, as with the inhibitors of the present invention.

For other articles describing previous efforts to devise renin inhibitors, see Marshall, Federation Proc. 35: 2494-2501, 1976; Burton et al., Proc. Natl. Acad. Sci. USA 77: 5476-5479, Sept. 1980; Suketa et al., Biochemistry 14: 3188, 1975; Swales, Pharmac. Ther. 7: 173-201, 1979; Kokubu et al., Nature 217: 456-457, Feb. 3, 1968; Matsushita et al., J. Antibiotics 28: 1016-1018, Dec. 1975; Lazar et al., Biochem. Pharma. 23: 2776-2778, 1974; Miller et al.,

Biohem. Pharma. 21: 2941-2944, 1972; Haber, Clinical Science 59:7s-19s, 1980; Rich et al., J. Org. Chem. 43: 3624, 1978, and J. Med. Chem. 23: 27, 1980; Burton et al., U.S. Pat. No. 4,269,827; Castro et al., U.S. Pat. No. 4,185,096; and Sankyo Jap. Pat. No. 76-067001.

## DETAILED DESCRIPTION OF THE INVENTION AND PREFERRED EMBODIMENTS

In accordance with the present invention there are provided renin inhibitory peptides of the formula:

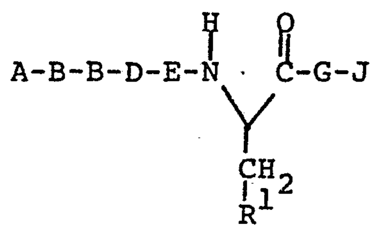

(I.)

wherein:

A is hydrogen; or 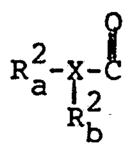

where

X is -O-; -O-CH-; -CH-O-; -CH-; -NH-CH-; or -S-CH-; and

$R_a^2$ and $R_b^2$ may be the same or different and are hydrogen; $W-(CH_2)_n-$ or $W-(CH_2)_m-CH=CH-(CH_2)_p$, where W is hydrogen; $C_{1-4}$alkyl; aryl; $C_{3-7}$cycloalkyl; or $C_{3-7}$cycloalkyl or aryl substituted with up to five members independently selected from the group consisting of $C_{1-8}$alkyl, trifluoromethyl, hydroxy, $C_{1-4}$alkoxy, and halo; n is 0 to 5; m is 0 to 2; and p is 0 to 2; except that where X is -O-, only one of $R_a^2$ or $R_b^2$ is present;

B is absent; glycyl; sarcosyl; or 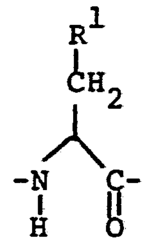 , where $R^1$ is as defined further below;

D is absent; or 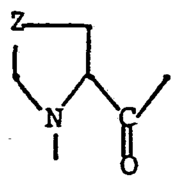 , where Z is $-(CH_2)_l-$ and l is 1 or 2; or -S-;

E is absent; or 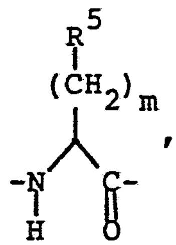 , where m is 1 to 4; and $R^5$ is hydrogen; $C_{1-4}$ alkyl; aryl; aryl-$C_{1-4}$ alkyl; aryl $C_{1-4}$ alkyl or aryl where the aryl portion is substituted with up to three members selected from the group consisting of $C_{1-4}$ alkyl, trifluoromethyl, hydroxy, $C_{1-4}$ alkoxy, and halo; or indolyl;

G is (1) 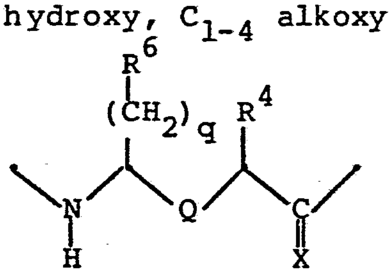 where q is 1 to 4; X is O, or H, H;

$R^4$ is hydrogen; or 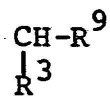 , where $R^9$ is hydrogen; $C_{1-4}$alkyl; hydroxy, or $C_{3-7}$cycloalkyl; and $R^3$ is hydrogen; $C_{1-4}$alkyl; aryl; aryl $C_{1-4}$alkyl; aryl $C_{1-4}$alkyl or aryl

substituted with up to three members selected from the group consisting of $C_{1-4}$alkyl, trifluoromethyl, hydroxy, $C_{1-4}$alkoxy, and halo; or indolyl;

$R^6$ is $C_{3-6}$ alkyl; $C_{3-7}$ cycloalkyl; aryl; or $C_{3-7}$cycloalkyl or aryl substituted with up to three members selected from the group consisting of $C_{1-4}$alkyl, trifluoromethyl, hydroxy, $C_{1-4}$alkoxy, and halo; and

Q is 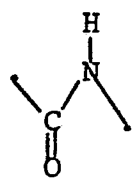 ; 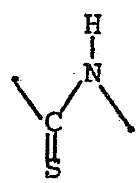 ; 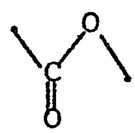 ; 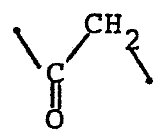 ;

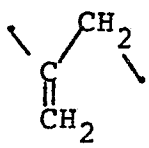 ; 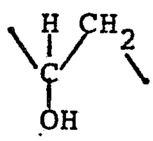 ; 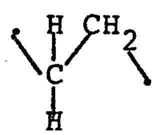 ; 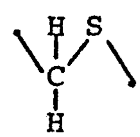 ;

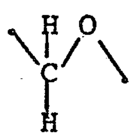 ; 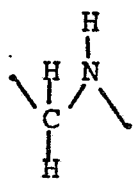 ; 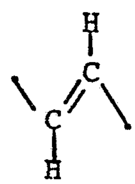 ; 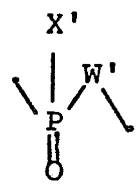 ;

wherein X' is hydroxy; amino; or mono- or di-$C_{1-4}$alkyl amino; and W' is absent; -O-; -NH-; or $-CH_2-$;

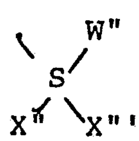

where X" and X"' are independently absent; or $\overset{O}{\overset{\uparrow}{S}}$; and

W" is absent; $-CH_2-$; or $-\overset{R^8}{\overset{|}{C}}H-$, where $R^8$ is hydrogen or $C_{1-3}$ alkyl;

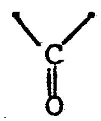 ; 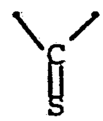 ; or

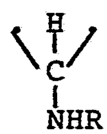 ; where R is hydrogen; $C_{1-4}$ alkyl; formyl; $C_{1-4}$ alkanoyl; aroyl; carboxy; $C_{1-4}$ alkoxycarbonyl; aryloxycarbonyl; or aryl $C_{1-4}$ alkoxycarbonyl; or

(2) 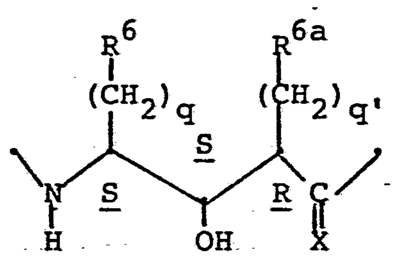 where q is 1 to 4; q' is 0 to 4; X is O or H, H;

$R^6$ is as defined above; and

$R^{6a}$ is hydrogen; $C_{1-8}$alkyl; $C_{2-8}$alkyl substituted with one or two members independently selected from the group consisting of hydroxy, carboxy, carboxy ester or amide, amino, mono-, di-, or tri-$C_{1-4}$alkylamino, and guanidyl; wherein said substitution occurs

on the last 1 or 2 carbon atoms of the alkyl chain; aryl; $C_{3-7}$cycloalkyl; or aryl or $C_{3-7}$cycloalkyl substituted with up to three members selected from the group consisting of $C_{1-4}$alkyl, trifluoromethyl, hydroxy, $C_{1-4}$alkoxy, and halo;
wherein the substituent of the above formula has 2<u>R</u>, 3<u>S</u>, 4<u>S</u> configuration;

J is (1) $-Y-(CH_2)_n-R^7$

where

Y is -NH- or -O-;

n is 0 to 5; and

$R^7$ is hydrogen, <u>provided</u> that where n is 0 and $R^7$ is hydrogen, that G is other than Sta and E is other than Phe; hydroxy; $C_{1-4}$alkyl; $C_{3-7}$cycloalkyl; aryl; aryl substituted with up to five members independently selected from the group consisting of $C_{1-6}$alkyl, trifluoromethyl, hydroxy, $C_{1-4}$alkoxy, amino, mono- or di- $C_{1-4}$ alkylamino, and halo; $N(R')_2$, where R' may be the same or different and is hydrogen, $C_{1-4}$alkyl, aryl, aryl $C_{1-4}$alkyl, heterocyclic, or heterocyclic $C_{1-4}$alkyl; $N(R')^{\oplus}{}_3A^{\ominus}$, where R' is as defined above, and $A^{\ominus}$ is a counterion; guanidyl; heterocyclic; heterocyclic substituted with up to five members independently selected from the group consisting of $C_{1-6}$alkyl, hydroxy, trifluoromethyl, $C_{1-4}$alkoxy, halo, aryl, aryl $C_{1-4}$alkyl, amino, and

mono- or di-$C_{1-4}$alkylamino; or heterocyclic substituted with another, the same or different, heterocyclic;

(2) 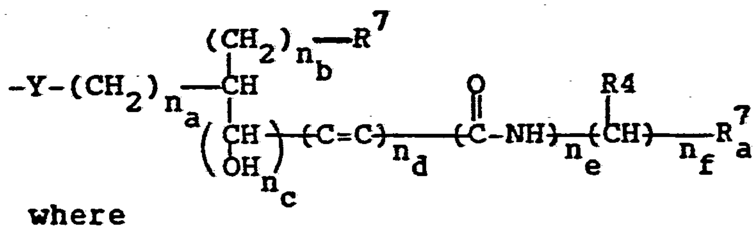

where

Y is as defined above;

$n_a$ is 0 or 1;

$n_b$ is 1 to 4;

$n_c$ is 0 or 1;

$n_d$ is 0 or 1;

$n_e$ is 0 or 1, provided that $n_e$ cannot be 1 when $n_d$ is 0;

$n_f$ is 1 to 4;

$R^4$ is hydrogen; or $-\underset{R^3}{CH}-R^9$, where

$R^9$ is hydrogen; $C_{1-4}$alkyl; hydroxy; or $C_{3-7}$cycloalkyl; and $R^3$ is hydrogen; $C_{1-4}$alkyl; aryl; aryl $C_{1-4}$alkyl; aryl $C_{1-4}$alkyl or aryl substituted with up to three members selected from the group consisting of $C_{1-4}$alkyl, trifluoromethyl, hydroxy, $C_{1-4}$alkoxy, and halo; or indolyl; and

$R^7$ and $R^7_a$ may be the same or different and have the same meaning as $R^7$ above and $R^7_a$ may additionally be

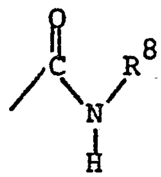 or 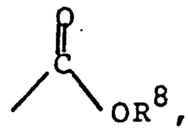,

where $R^8$ is hydrogen or $C_{1-3}$alkyl;

(3) 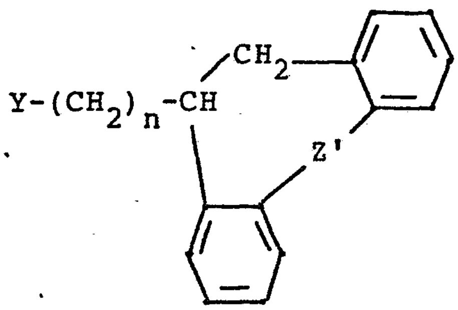

where

Y is as defined above;

n is 0 or 1; and

Z' is

(a) $-(CH_2)_n-\underset{R^8}{\underset{|}{CH}}-$

where

n is 0 or 1; and

$R^8$ is as defined above; or

(b) $-(CH_2)_n-\underset{CH_2}{\underset{\|}{C}}-$

where

n is 0 or 1; or

(4) (a) $Y-(\overset{R^{10}}{\overset{|}{C}}H)_q-R^{11}$; (b) $Y-(\overset{R^{12}}{\overset{|}{C}}H)_{q'}-R^{13}$; or

(c) $Y-CH(R^{14})-R^{11}$

where

Y is -NH- or -O-;

q is 1-5;

q' is 0-5;

$R^{10}$ is hydrogen; hydroxy; $N(R")_2$, where R" may be the same or different and is hydrogen or $C_{1-4}$alkyl; guanidyl; or $N^{\oplus}(R")_3A^{\ominus}$, where R" is as defined above, and $A^{\ominus}$ is a counterion; provided that at least one $R^{10}$ is not hydrogen;

$R^{11}$ is $C_{1-4}$alkyl; $C_{3-7}$cycloalkyl; aryl; aryl substituted with up to three members independently selected from the group consisting of $C_{1-6}$alkyl, trifluoromethyl, hydroxy, $C_{1-4}$alkoxy, amino, mono- or di- $C_{1-4}$alkylamino, amino $C_{1-4}$alkyl, mono-, di-, or tri-$C_{1-4}$alkylamino-$C_{1-4}$alkyl, halo, carboxy, carboxy ester or amide, carboxy-$C_{1-4}$-alkoxy, carboxy-$C_{1-4}$-alkoxy ester or amide, α-aminocarboxy-$C_{1-4}$alkyl, α-aminocarboxy-$C_{1-4}$-alkyl ester or amide, carboxy-$C_{1-4}$-alkyl, carboxy-$C_{1-4}$-alkyl ester or amide, guanidyl, and guanidyl-$C_{1-4}$-alkyl; carboxy, ester or amide; sulfo; heterocyclic; or heterocyclic substituted with up to five members independently selected from the group

consisting of $C_{1-6}$alkyl, hydroxy, trifluoromethyl, $C_{1-4}$alkoxy, halo, aryl, aryl $C_{1-4}$alkyl, amino, and mono- or di-$C_{1-4}$alkylamino;

$R^{12}$ is hydrogen; or carboxy, ester or amide;

$R^{13}$ is carboxy, ester or amide; sulfo; or aryl substituted with up to three members selected from the group consisting of amino-$C_{1-4}$alkyl, mono-, di-, or tri-$C_{1-4}$-alkylamino-$C_{1-4}$-alkyl, halo, carboxy, carboxy ester or amide, carboxy-$C_{1-4}$alkoxy, carboxy-$C_{1-4}$alkoxy ester or amide, α-amino-carboxy-$C_{1-4}$alkyl, α-aminocarboxy-$C_{1-4}$alkyl ester or amide, carboxy-$C_{1-4}$alkyl, carboxy-$C_{1-4}$alkyl ester or amide, guanidyl, and guanidyl-$C_{1-4}$alkyl; and

$R^{14}$ is carboxy, ester or amide;

(d) 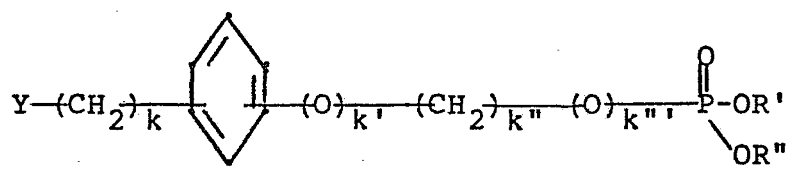 ; or

(e) 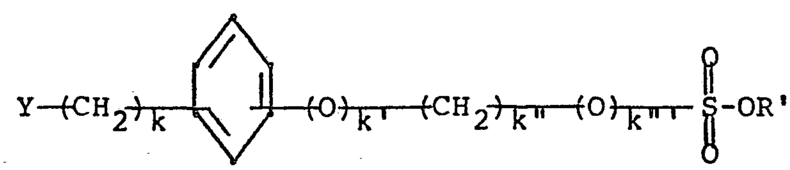 ,

where

Y is -NH- or -O-;

k is 0-4;

k' is 0 or 1;

k" is 0-4;

k"'is 0 or 1;

R' is hydrogen or $C_{1-4}$alkyl; and

R" is hydrogen or $C_{1-4}$alkyl;

$R^1$ is hydrogen; $C_{1-4}$ alkyl; hydroxy $C_{1-4}$alkyl; aryl; aryl substituted with up to three members selected from the group consisting of $C_{1-4}$ alkyl, trifluoromethyl, hydroxy, $C_{1-4}$ alkoxy, and halo; indolyl; 4-imidazolyl; amino $C_{2-4}$ alkyl; acyl $C_{2-4}$ alkyl wherein the acyl is $R^9-\overset{O}{\overset{\|}{C}}-$ and $R^9$ is as defined above; guanidyl $C_{2-3}$ alkyl; or methylthiomethyl;

wherein all of the asymmetric carbon atoms have an S configuration, except for those in the B, D, and G substituents, which may have an S or R configuration;

and a pharmaceutically acceptable salt thereof.

While both the S and R chiralities for asymmetric carbon atoms in the B substituent are included in the peptides of the present invention, preferred chiralities are indicated in the description which follows.

In the above definitions, the term "alkyl" is intended to include both branched and straight chain hydrocarbon groups having the indicated number of carbon atoms.

The term "halo" means fluoro, chloro, bromo and iodo.

The aryl substituent represents phenyl, and naphthyl.

The heterocyclic substituent recited above represents any 5- or 6-membered ring containing from one to three heteroatoms selected from the group consisting of nitrogen, oxygen, and sulfur; having various degrees of unsaturation; wherein the nitrogen and sulfur heteroatoms may optionally be oxidized; wherein the nitrogen heteroatom may optionally be quaternized; and including any bicyclic group in which any of the above heterocyclic rings is fused to a benzene ring. Heterocyclic substituents in which nitrogen is the heteroatom are preferred, and of t: those containing a single nitrogen atom are preferred. Fully saturated heterocyclic substituents are also preferred. Thus, piperidine is a preferred heterocyclic substituent. Other preferred heterocyclic substituents are: pyrryl, pyrrolinyl, pyrrolidinyl, pyrazolyl, pyrazolinyl, pyrazolidinyl, imidazolyl, imidazolinyl, imidazolidinyl, pyridyl, piperidinyl, pyrazinyl, piperazinyl, pyrimidinyl, pyridazinyl, oxazolyl, oxazolidinyl, isoxazolyl, isoxazolidinyl, morpholinyl, thiazolyl, thiazolidinyl, isothiazolyl, isothiazolidinyl, indolyl, quinolinyl, isoquinolinyl, benzimidazolyl, benzothiazolyl, benzoxazolyl, furyl, thienyl and benzothienyl.

Where the heterocyclic substituent itself is substituted, it is preferred that the substituent be aryl$C_{1-4}$alkyl.

The novel renin inhibitory peptides of the present invention may also be described in terms of common amino acid components and closely related analogs thereof, in accordance with the following formula:

A-B-B-D-E-Y-G-J-

(II.)

The A, B, D, G, J and L components correspond to the same portions of Formula I.

The common amino acid components of Formula II are as follows:

A has the same meaning as above in Formula I;

B is Ala, Leu, Ser, Thr, Phe, Tyr, Trp, His, Lys, Orn, Arg, or Met;

D is Pro;

E is Ala, Leu, Phe, HomoPhe, BisHomoPhe, Tyr, HomoTyr, Trp, or HomoTrp;

Y is the same as B;

G has the same meaning as above in Formula I; and

J has the same meaning as above in Formula I;

It will be understood that closely related analogs of the above common amino acids, for example, aliphatic amino acids in addition to Ala, Val, Leu, and Ile, such as α-aminobutyric acid (Abu), and substituted phenyl derivatives of Phe, are included in the broad description of the novel inhibitory peptides of the present invention represented by Formula I and its definitions. Thus, the peptides of Formula II and its definitions represent preferred peptides of the present invention.

Preferred inhibitory peptides of the present invention are the following:

BOC[1]-His-Pro-Phe-His-Sta-OEt

BOC-Phe-His-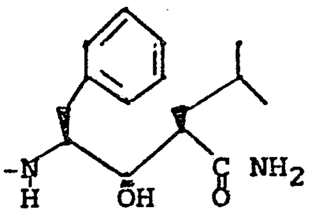

BOC-Phe-His-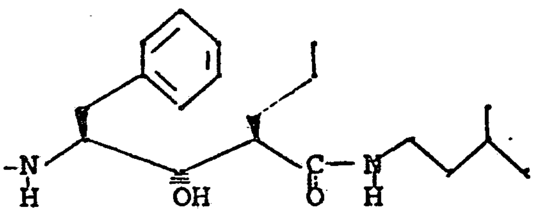

BOC-Phe-His-ACHPA[2]-$NH_2$

BOC-HomoPhe-His-Sta-$NH_2$

BOC-Phe-His-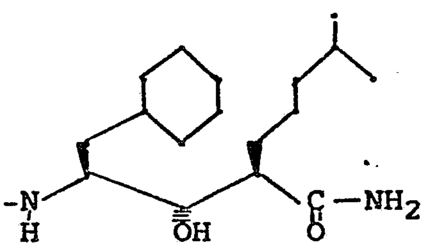

BOC-Phe-His-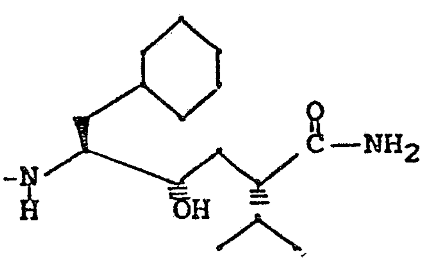

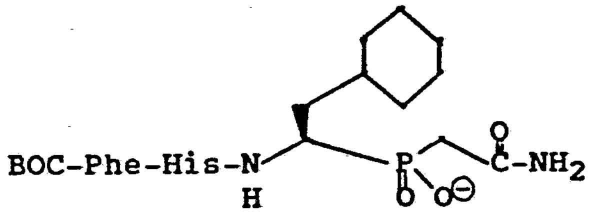

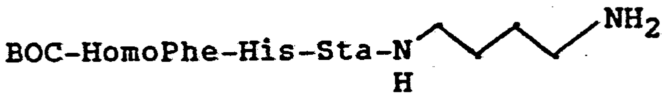

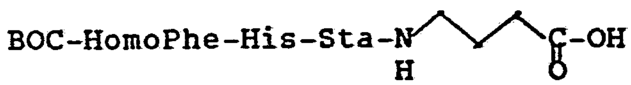

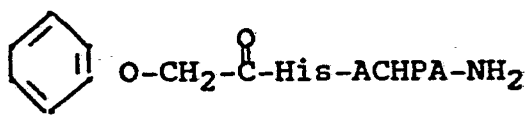

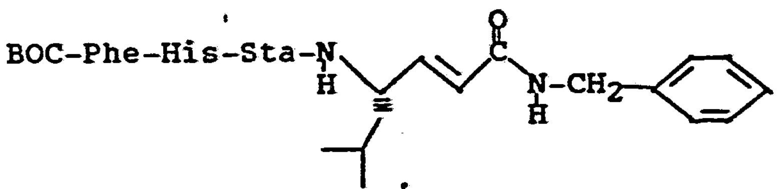

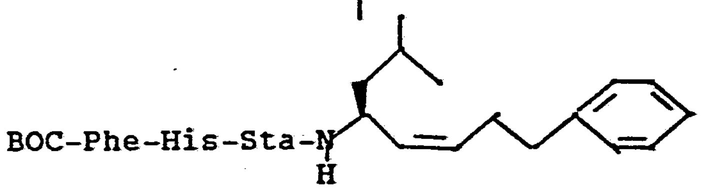

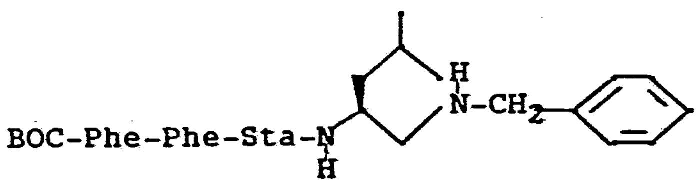

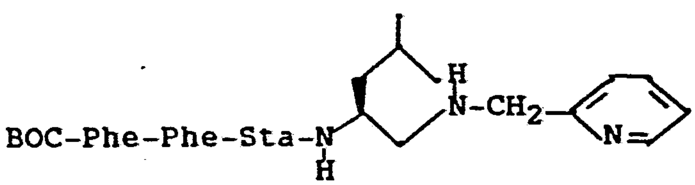

BOC-Phe-Phe-Sta-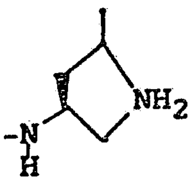

BOC-Phe-Phe-Sta-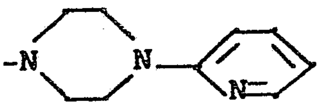

BOC-Phe-Phe-Sta-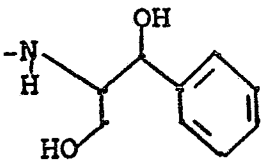

BOC-Phe-Phe-Sta-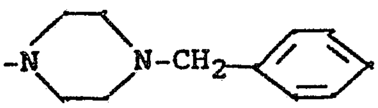

BOC-Phe-His-Sta-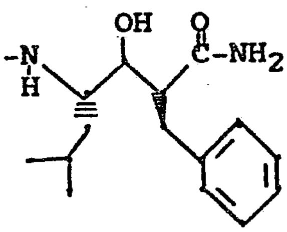

BOC-Phe-Phe-Sta-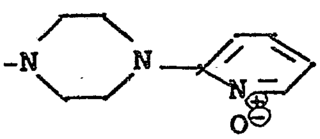

BOC-Phe-His-AHPPA[3]-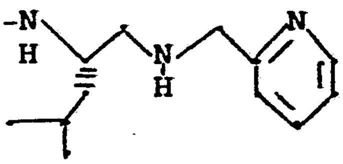

---

[1] BOC = *Tert*-butyloxycarbonyl.

[2] ACHPA = (3S, 4S)-4-amino-5-cyclohexyl-3-hydroxy-pentanoyl.

[3] AHPPA = (3S, 4S)-4-amino-3-hydroxy-5-phenyl-pentanoyl.

The inhibitory peptides of the present invention may be better appreciated in terms of substrate analogy from the following illustration of Formula I alongside the octapeptide sequence of a portion of the *pig* renin substrate, which renin cleaves between $Leu^{10}$ and $Leu^{11}$:

| | | | | | | | |
|---|---|---|---|---|---|---|---|
| Pro | Phe | His | Leu | Leu | Val | Tyr | |
| 7 | 8 | 9 | 10 | (11) | 12 | 13 | (14) |

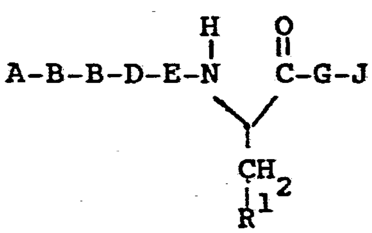

(I.)

As can be seen, a unique aspect and essential feature of the present invention is the substitution of the G component for the double amino acid

sequence: $Leu^{10}$-$Leu^{11}$ in the endogenous pig renin substrate. It is believed that substitution of this component for both leucine amino acids rather than just one leucine results in an improved substrate analogy due to the greater linear extent of the component as compared to a single leucine component. Thus, the component more closely approximates Leu-Leu in linear extent, and thereby provides a better "fit" to the renin enzyme.

The inhibitory peptides of the present invention may also be better appreciated in terms of substrate analogy from the following illustration of Formula I alongside the octapeptide sequence of a portion of the human renin substrate, which renin cleaves between $Leu^{10}$ and $Val^{11}$:

| | | | | | | | |
|---|---|---|---|---|---|---|---|
| Pro | Phe | His | Leu | Val | Ile | His | |
| 7 | 8 | 9 | 10 | (11) | 12 | 13 | (14) |

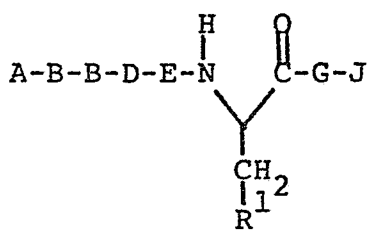

(I.)

As can be seen, a unique aspect and essential feature of the present invention is the substitution of the G component for the double amino acid sequence: $Leu^{10}$-$Val^{11}$ in the endogenous human renin substrate. It is believed that substitution of this component for both the leucine and valine amino acids rather than just the leucine results in an improved substrate analogy due to the greater linear extent of the

greater linear extent of the component as compared to a single leucine component. Thus, the component more closely approximates Leu-Val in linear extent, and thereby provides a better "fit" to the human renin enzyme.

The Formula I compounds can be used in the form of salts derived from inorganic or organic acids and bases. Included among such acid addition salts are the following: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups can be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others. Water or oil-soluble or dispersible products are thereby obtained.

The present invention is also directed to combinations of the novel renin-inhibitory peptides of Formula I with one or more antihypertensive agents selected from the group consisting of diuretics, α and/or β-adrenergic blocking agents, CNS-acting agents, adrenergic neuron blocking agents, vasodilators, angiotensin I converting enzyme inhibitors, calcium channel blockers, and other antihypertensive agents.

For example, the compounds of this invention can be given in combination with such compounds or salt or other derivative forms thereof as:

Diuretics: acetazolamide; amiloride; bendroflumethiazide; benzthiazide; bumetanide; chlorothiazide; chlorthalidone; cyclothiazide; ethacrynic acid; furosemide; hydrochlorothiazide; hydroflumethiazide; indacrinone (racemic mixture, or as either the (+) or (-) enantiomer alone, or a manipulated ratio, e.g., 9:1 of said enantiomers, respectively); metolazone; methyclothiazide; muzolimine; polythiazide; quinethazone; sodium ethacrynate; sodium nitroprusside; spironolactone; ticrynafen; triamterene; trichlormethiazide;

α-Adrenergic Blocking Agents: dibenamine; phentolamine; phenoxybenzamine; prazosin; tolazoline;

β-Adrenergic Blocking Agents: atenolol; metoprolol; nadolol; propranolol; timolol;
(($\pm$)-2-[3-(tert-butylamino)-2-hydroxypropoxy]-2-furananilide) (ancarolol);
(2-acetyl-7-(2-hydroxy-3-isopropylaminopropoxy)benzofuran HCl) (befunolol);

((±)-1-(isopropylamino)-3-(p-(2-cyclopropylmethoxyethyl)-phenoxy)-2-propranol HCl) (betaxolol);

(1-[(3,4-dimethoxyphenethyl)amino]-3-(m-tolyloxy)-2-propanol HCl) (bevantolol);

(((±)-1-(4-((2-isopropoxyethoxy)methyl)phenoxy)-3-isopropylamino-2-propanol)fumarate) (bisoprolol);

(4-(2-hydroxy-3-[4-(phenoxymethyl)-piperidino]-propoxy)-indole);

(carbazolyl-4-oxy-5,2-(2-methoxyphenoxy)-ethylamino-2-propanol);

(1-((1,1-dimethylethyl)amino)-3-((2-methyl-1H-indol-4-yl)oxy)-2-propanol benzoate) (bopindolol);

(1-(2-exobicyclo[2.2.1]-hept-2-ylphenoxy)-3-[(1-methylethyl)-amino]-2-propanol HCl) (bornaprolol);

(o-[2-hydroxy-3-[(2-indol-3-yl-1,1-dimethylethyl)-amino]propoxy]benzonitrile HCl) (bucindolol);

(α-[(tert.butylamino)methyl]-7-ethyl-2-benzofuranmethanol) (bufuralol);

(3-[3-acetyl-4-[3-(tert.butylamino)-2-hydroxypropyl]-phenyl]-1,1-diethylurea HCl) (celiprolol);

((±)-2-[2-[3-[(1,1-dimethylethyl)amino]-2-hydroxy-propoxy]phenoxy]-N-methylacetamide HCl) (cetamolol);

(2-benzimidazolyl-phenyl(2-isopropylaminopropanol));

((±)-3'-acetyl-4'-(2-hydroxy-3-isopropylaminopropoxy)-acetanilide HCl) (diacetolol);

(methyl-4-[2-hydroxy-3-[(1-methylethyl)aminopropoxy]]-benzenepropanoate HCl) (esmolol);

(erythro-DL-1-(7-methylindan-4-yloxy)-3-isopropylaminobutan-2-ol);

(1-(tert.butylamino)-3-[O-(2-propynyloxy)phenoxy]-2-propanol (pargolol);

(1-(tert.butylamino)-3-[o-(6-hydrazino-3-pyridazinyl)-phenoxy]-2-propanol diHCl) (prizidilol);

((-)-2-hydroxy-5-[(R)-1-hydroxy-2-[(R)-(1-methyl-3-phenylpropyl)amino]ethyl]benzamide);

(4-hydroxy-9-[2-hydroxy-3-(isopropylamino)-propoxy]-7-methyl-5H-furo[3,2-g][1]-benzopyran-5-one) (iprocrolol);

((-)-5-(tert.butylamino)-2-hydroxypropoxy]-3,4-dihydro-1-(2H)-naphthalenone HCl) (levobunolol);

(4-(2-hydroxy-3-isopropylamino-propoxy)-1,2-benzisothiazole HCl);

(4-[3-(tert.butylamino)-2-hydroxypropoxy]-N-methylisocarbostyril HCl);

((±)-N-2-[4-(2-hydroxy-3-isopropyl aminopropoxy)-phenyl]ethyl-N'-isopropylurea) (pafenolol);

(3-[[(2-trifluoroacetamido)ethyl]amino]-1-phenoxy-propan-2-ol);

(N-(3-(o-chlorophenoxy)-2-hydroxypropyl)-N'-(4'-chloro-2,3-dihydro-3-oxo-5-pyridazinyl)ethylenediamine);

((±)-N-[3-acetyl-4-[2-hydroxy-3-[(1-methylethyl)amino]-propoxy]phenyl]butanamide) (acebutolol);

((±)-4'-[3-(tert-butylamino)-2-hydroxypropoxy]spiro-[cyclohexane-1,2'-indan]-1'-one) (spirendolol);

(7-[3-[[2-hydroxy-3-[(2-methylindol-4-yl)oxy]propyl]-amino]butyl]thiophylline) (teoprolol);

((±)-1-tert.butylamino-3-(thiochroman-8-yloxy)-2-propanol) (tertatolol);

((±)-1-tert.butylamino-3-(2,3-xylyloxy)-2-propanol HCl) (xibenolol);

(8-[3-(tert.butylamino)-2-hydroxypropoxy]-5-methyl-coumarin) (bucumolol);

(2-(3-(tert.butylamino)-2-hydroxy-propoxy)benzonitrile HCl) (bunitrolol);

((±)-2'-[3-(tert-butylamino)-2-hydroxypropoxy-5'-fluorobutyrophenone) (butofilolol);

(1-(carbazol-4-yloxy)-3-(isopropylamino)-2-propanol) (carazolol);

(5-(3-tert.butylamino-2-hydroxy)propoxy-3,4-dihydrocarbostyril HCl) (carteolol);

(1-(tert.butylamino)-3-(2,5-dichlorophenoxy)-2-propanol) (cloranolol);

(1-(inden-4(or 7)-yloxy)-3-(isopropylamino)-2-propanol HCl) (indenolol);

(1-isopropylamino-3-[(2-methylindol-4-yl)oxy]-2-propanol) (mepindolol);

(1-(4-acetoxy-2,3,5-trimethylphenoxy)-3-isopropylaminopropan-2-ol) (metipranolol);

(1-(isopropylamino)-3-(o-methoxyphenoxy)-3-[(1-methylethyl)amino]-2-propanol) (moprolol);

((1-tert.butylamino)-3-[(5,6,7,8-tetrahydro-cis-6,7-dihydroxy-1-naphthyl)oxy]-2-propanol) (nadolol);

((S)-1-(2-cyclopentylphenoxy)-3-[(1,1-dimethylethyl)-amino]-2-propanol sulfate (2:1)) (penbutolol);

(4'-[1-hydroxy-2-(amino)ethyl]methanesulfonanilide) (sotalol);

(2-methyl-3-[4-(2-hydroxy-3-tert.butylaminopropoxy)-phenyl]-7-methoxy-isoquinolin-1-(2H)-one);

(1-(4-(2-(4-fluorophenyloxy)ethoxy)phenoxy)-3-isopropylamino-2-propanol HCl);

((-)-p-[3-[(3,4-dimethoxyphenethyl)amino]-2-hydroxypropoxy]-ß-methylcinnamonitrile) (pacrinolol);

((±)-2-(3'-tert.butylamino-2'-hydroxypropylthio)-4-(5'-carbamoyl-2'-thienyl)thiazole HCl) (arotinolol);

((±)-1-[p-[2-(cyclopropylmethoxy)ethoxy]phenoxy]-3-(isopropylamino)-2-propanol) (cicloprolol);

((±)-1-[(3-chloro-2-methylindol-4-yl)oxy]-3-[(2-phenoxyethyl)amino]-2-propanol) (indopanolol);

((±)-6-[[2-[[3-(p-butoxyphenoxy)-2-hydroxypropyl]-amino]ethyl]amino]-1,3-dimethyluracil) (pirepolol);

(4-(cyclohexylamino)-1-(1-naphtholenyloxy)-2-butanol);

(1-phenyl-3-[2-[3-(2-cyanophenoxy)-2-hydroxypropyl]-aminoethyl]hydantoin HCl);

(3,4-dihydro-8-(2-hydroxy-3-isopropylaminopropoxy)-3-nitroxy-2H-1-benzopyran) (nipradolol);

α and ß-Adrenergic Blocking Agents:

((±)-1-tert-butylamino)-3-[o-[2-(3-methyl-5-isoxazolyl)vinyl]phenoxy]-2-propanol) (isoxaprolol);

(1-isopropylamino-3-(4-(2-nitroxyethoxy)phenoxy)-2-propanol HCl);

(4-hydroxy-α-[[3-(4-methoxyphenyl)-1-methylpropyl]-aminomethyl]-3-(methylsulfinyl)-benzmethanol HCl) (sulfinalol);

(5-[1-hydroxy-2-[[2-(o-methoxyphenoxy)ethyl]amino]-ethyl]-2-methylbenzenesulfonamide HCl);

(5-[1-hydroxy-2-[(1-methyl-3-phenylpropyl)amino]ethyl]-salicylamide HCl) (labetalol);

(1-((3-chloro-2-methyl-1H-indol-4-yl)oxy)-3-((2-phenoxyethyl)amino)-2-propanol-hydrogenmalonate) (ifendolol);

(4-(2-hydroxy-3-[(1-methyl-3-phenylpropyl)amino]-propoxy)benzeneacetamide);

(1-[3-[[3-(1-naphthoxy)-2-hydroxypropyl]-amino]-3,3-dimethyl-propyl]-2-benzimidazolinone);

(3-(1-(2-hydroxy-2-(4-chlorophenylethyl)-4-piperidyl)-3,4-dihydroxy)quinoxolin-2(1H)-one);

CNS-Acting Agents: clonidine; methyldopa;

Adrenergic Neuron Blocking Agents: guanethidine; reserpine and other rauwolfia alkaloids such as rescinnamine;

Vasodilators: diazoxide; hydralazine; minoxidil;

Angiotensin I Converting Enzyme Inhibitors:
1-(3-mercapto-2-methyl-1-oxopropyl)-L-proline (captopril);
(1-(4-ethoxycarbonyl-2,4(R,R)-dimethylbutanoyl)-indoline-2(S)-carboxylic acid);
(2-[2-[[1-(ethoxycarbonyl)-3-phenyl-propyl]amino]-1-oxopropyl]-1,2,3,4-tetrahydro-3-isoquinoline carboxylic acid);
((S)-1-[2-[[1-(ethoxycarbonyl)-3-phenylpropyl]amino]-1-oxopropyl]octahydro-1H-indole-2-carboxylic acid HCl);
(N-cyclopentyl-N-(3-(2,2-dimethyl-1-oxopropyl)thiol-2-methyl-1-oxopropyl)glycine) (pivalopril);
((2R,4R)-2-(2-hydroxyphenyl)-3-(3-mercaptopropionyl)-4-thiazolidinecarboxylic acid);
(1-(N-[1(S)-ethoxycarbonyl-3-phenylpropyl]-(S)-alanyl)-cis,syn-octahydroindol-2(S)-carboxylic acid HCl);
((-)-(S)-1-[(S)-3-mercapto-2-methyl-1-oxopropyl]-indoline-2-carboxylic acid);
([1(S),4S]-1-[3-(benzoylthio)-2-methyl-1-oxopropyl]-4-phenylthio-L-proline;
(3-([1-ethoxycarbonyl-3-phenyl-(1S)-propyl]amino)-2,3,4,5-tetrahydro-2-oxo-1-(3S)-benzazepine-1-acetic acid HCl);
(N-(2-benzyl-3-mercaptopropanoyl)-S-ethyl-L-cysteine) and the S-methyl analogue;

(N-(1(S)-ethoxycarbonyl-3-phenylpropyl)-L-alanyl-L-proline maleate) (enalapril);

N-[1-(S)-carboxy-3-phenylpropyl]-L-alanyl-1-proline;

$N^2$-[1-(S)-carboxy-3-phenylpropyl]-L-lysyl-L-proline (lysinopril);

<u>Calcium Channel Blockers:</u>

α-[3-[[2-(3,4-dimethoxyphenyl)ethyl]methylamino]-propyl]-3,4-dimethoxy-α-(1-methylethyl)benzene-acetonitrile (verapamil);

1,4-dihydro-2,6-dimethyl-4-(2-nitrophenyl)-3,5-pyridinedicarboxylic acid dimethyl ester (nifedipine);

2-(2,2-dicyclohexylethyl)piperidine (perhexiline);

N-(1-methyl-2-phenylethyl)- -phenylbenzenepropanamine (prenylamine);

3-(aminosulfonyl)-4-chloro-N-(2,3-dihydro-2-methyl-1H-indol-1-yl)benzamide (indapamide);

(2'-(2-diethylaminoethoxy)-3-phenylpropiophenone (etafenone);

(4-[4,4-bis-(4-fluorophenyl)butyl]-N-(2,6-dimethylphenyl)-1-piperazineacetamide) (lidoflazine);

(2-(N-benzyl-N-methylamino)ethylmethyl-2,6-dimethyl-4-(m-nitrophenyl)-1,4-dihydro-3,5-pyridinedicar-boxylate HCl) (nicardipine);

(N-(3,4-dimethoxyphenethyl)-2-(3,4-dimethoxyphenyl)-N-methyl-m-dithiane-2-propylamine-1,1,3,3-tetraoxide) (tiapamil);

(5,6-dimethoxy-2-(3-[(α-(3,4-dimethoxy)phenylethyl)-methylamino]propyl)phthalimidine) (falipamil);

(ß-[(2-methylpropoxy)methyl]-N-phenyl-N-phenylmethyl-1-pyrrolidineethanamine HCl monohydrate) (bepridil);

((+)-cis-3-(acetyloxy)-5-[2-(dimethylamino)ethyl]-2,3-dihydro-2-(4-methoxyphenyl)-1,5-benzothiazepin-4-(5H)-one) (diltiazem);

((E)-1-[bis-(p-fluorophenyl)methyl]-4-cinnamylpiperazine di HCl) (flunarizine);

(5-[(3,4-dimethoxyphenethyl)methylamino]-2-isopropyl-2-(3,4,5-trimethoxyphenyl)valeronitrile (gallopamil);

(ethylmethyl(2,3-dichlorophenyl)-1,4-dihydro-2,6-dimethyl-3,5-pyridinedicarboxylate (felodipine);

(isopropyl-2-methoxyethyl-1,4-dihydro-2,6-dimethyl-4-(3-nitrophenyl)-3,5-pyridinecarboxylate) (nimodipine);

(3-ethyl-5-methyl-1,4-dihydro-2,6-dimethyl-4-(3-nitrophenyl)-3,5-pyridine-dicarboxylate) (nitrendipine);

<u>Other Antihypertensive Agents</u>: aminophylline; cryptenamine acetates and tannates; deserpidine; meremethoxylline procaine; pargyline; trimethaphan camsylate;

and the like, as well as admixtures and combinations thereof.

Typically, the individual daily dosages for these combinations can range from about one-fifth of the minimally recommended clinical dosages to the maximum recommended levels for the entities when they are given singly. Coadministration is most readily accomplished by combining the active ingredients into a suitable unit dosage form containing the proper dosages of each. Other methods of coadministration are, of course, possible.

The novel peptides of the present invention possess an excellent degree of activity in treating renin-associated hypertension and hyperaldosteronism.

For these purposes the compounds of the present invention may be administered parenterally, by inhalation spray, or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as mice, rats, horses, dogs, cats, etc., the compounds of the invention are effective in the treatment of humans.

The pharmaceutical compositions may be in the form of a sterile injectable preparation, for example as a sterile injectable aqueous or oleagenous suspension. This suspension may be formulated according to the known art using suitable dispersing or wetting agents and suspending agents. The sterile injectable preparation may also be a sterile injectable solution or suspension in a non-toxic parenterally-acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectibles.

The peptides of this invention may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials are cocoa butter and polyethylene glycols.

Dosage levels of the order of 0.1 to 4.0 grams per day are useful in the treatment of the above indicated conditions. For example, renin-associated hypertension and hyperaldosteronism are effectively treated by the administration of from 1.0 to 50 milligrams of the compound per kilogram of body weight per day.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

Thus, in accordance with the present invention there is further provided a pharmaceutical composition for treating renin-associated hypertension and hyperaldosteronism, comprising a pharmaceutical carrier and a therapeutically effective amount of a peptide of the formula:

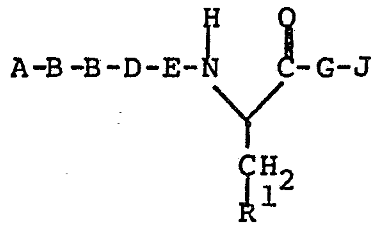

(I.)

wherein A, B, D, E, $R^1$, G, and J have the same meaning as recited further above for Formula I; wherein all of the asymmetric carbon atoms have an S configuration, except for those in the B, D, and G substituents, which may have an S or R configuration; and a pharmaceutically acceptable salt thereof.

Also, in accordance with the present invention there is still further provided a method of treating renin-associated hypertension and hyperaldosteronism, comprising administering to a patient in need of such treatment, a therapeutically effective amount of a peptide of the formula:

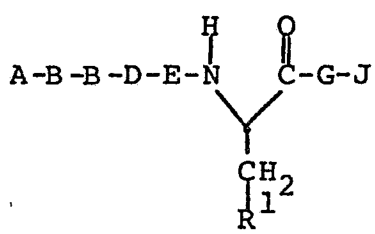

(I.)

wherein A, B, D, E, $R^1$, G, and J have the same meaning as recited further above for Formula I; wherein all of the asymmetric carbon atoms have an S configuration, except for those in the A, D, and G substituents, which may have an S or R configuration; and a pharmaceutically acceptable salt thereof.

The renin-inhibitory novel peptides of the present invention may also be utilized in diagnostic methods for the purpose of establishing the significance of renin as a causative or contributory factor in hypertension or hyperaldosteronism in a particular patient. For this purpose the novel peptides of the present invention may be administered in a single dose of from 0.1 to 10 mg per kg of body weight.

Both in vivo and in vitro methods may be employed. In the in vivo method, a novel peptide of the present invention is administered to a patient, preferably by intravenous injection, although parenteral administration is also suitable, at a hypotensive dosage level and as a single dose, and there may result a transitory fall in blood pressure. This fall in blood pressure, if it occurs, indicates supranormal plasma renin levels.

An in vitro method which may be employed involves incubating a body fluid, preferably plasma, with a novel peptide of the present invention and, after deproteinization, measuring the amount of angiotensin II produced in nephrectomized, pentolinium-treated rats. Another in vitro method involves mixing the plasma or other body fluid with a novel peptide of the present invention and injecting the mixture into a test animal. The difference in pressor response with and without added peptide is a measure of the renin content of the plasma.

Pepstatin may be employed in the methods described above as an active control. See, e.g., U.S. Patent Nos. 3,784,686 and 3,873,681 for a description of the use of pepstatin in diagnostic methods of this type.

The novel peptides of the present invention may be prepared in accordance with well-known procedures for preparing peptides from their constituent amino acids, which will be described in more detail below.

A general method of preparation may be described in the following terms:

A method of preparing a peptide of Formula I, said peptide being comprised of from two to six amino acids identified as I through VI, amino acid (AA) I being the component G at the C-terminus of said peptide, and amino acid (AA) VI being at the N-terminus of said peptide, to which substituent A is attached, comprising the steps of:

(A) treating the desired ester or amide of the C-terminus amino acid (AA I) with the next adjacent amino acid (AA II) of said peptide, the amino group of said amino acid being protected by a protecting group, in the presence of a condensing agent, whereby a dipeptide of the two amino acids (AA I and II) is formed;

(B) deprotecting the dipeptide formed in Step (A) by removing the protecting group from the amino group of AA II, to give the peptide of Formula I wherein A is hydrogen;

(C) treating the dipeptide formed in Step (B) where an ester of AAI is employed with hydrazine to give the corresponding hydrazide, followed by treatment of said hydrazide with acidic nitrite to give the corresponding acyl azide, followed by treatment of said acyl azide with the appropriate amine compound to give the desired J substituent in the peptide of Formula I; and optionally

(D) treating the dipeptide formed in Step (C) with

$$R^2_a-\underset{R^2_b}{\underset{|}{X}}-\overset{\overset{O}{\|}}{C}-W,$$ where X, $R^2_a$, and $R^2_b$, are as defined above and W is an acid halide, anhydride, or other carbonyl activating group, to give the peptide of Formula I wherein A is other than hydrogen; and optionally

(E) forming a tripeptide up to a hexapeptide of AA I, through AA VI, by repeating the procedures of Steps (A) and (B) using protected AA III through protected AA VI;

(F) deprotecting the tripeptide through hexapeptide formed in Step (E) to give the peptide of Formula I wherein A is hydrogen; and optionally

(G) treating the ttipeptide through hexapeptide formed in Step (H) with $R^2_a-\underset{R^2_b}{\underset{|}{X}}-\overset{\overset{O}{\|}}{C}-W$, where X, $R^2_a$, and $R^2_b$ are as defined above and W is an acid halide, anhydride, or other carboxyl activating group, to give the peptide of Formula I wherein A is other than hydrogen;

said method also comprising, where necessary, protection of sidechain substituents of the component amino acids AA I through AA VI, with deprotection being carried out as a final step; said method also comprising any combination of the steps set out above, whereby the amino acids I through VI and substituents A, G, and J, are assembled in any desired order to prepare the peptide of Formula I; and said method also comprising employment of the steps set out above in a solid phase sequential

synthesis, whereby in the initial step the carboxyl group of the selected amino acid is bound to a synthetic resin substrate while the amino group of said amino acid is protected, followed by removal of the protecting group, the succeeding steps being as set out above, the peptide as it is assembled being attached to said synthetic resin substrate; followed by a step of removing the peptide of Formula I from said synthetic resin substrate; and after removal of the peptide of Formula I from said synthetic resin substrate, the step of teating said ester thereof in accordance with the procedures described in Step (C) above to give the desired J substituent in the peptide of Formula I; removal of sidechain protecting groups being accomplished either before or after removal of the peptide of Formula I from said synthetic resin substrate.

Preparation of the peptides of Formula I having the desired J substituent, as described above in Step (C), may be illustrated as follows for the particular case where J=benzylamide (and PEP represents the remaining portion of the peptide of Formula I):

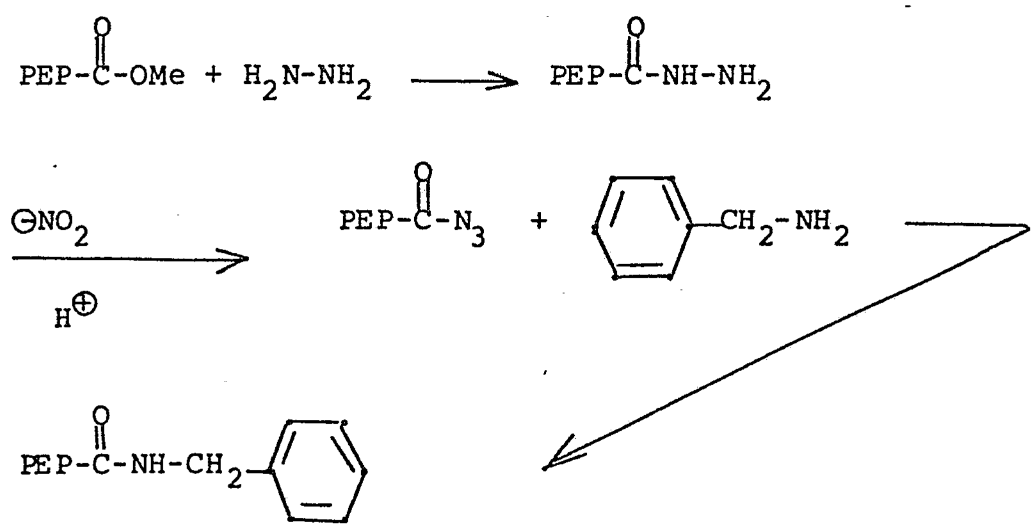

The G Component

I. Where the Component G Is a Peptide Isostere: the novel peptides of the present invention may be prepared in accordance with well-known procedures in synthetic chemistry, as is described in more detail further below. Attachment of the isostere component G to the other components of the novel peptides of the present invention is carried out in the same manner as for any of said other components, and may involve addition of the isostere component in a protected form. For example, the following reactive groups would require such protection:

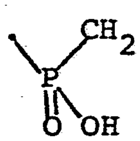 as 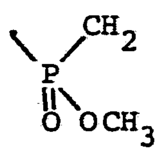 ; and

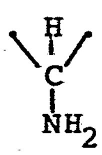 as 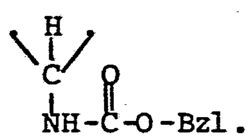

Such protecting groups may be removed as a final or near final step, for example, by base hydrolysis in the former case, or by hydrogenation in the latter.

Preparation of the particular isostere components may be carried out in accordance with procedures described below and in the literature cited particularly as follows:

A. 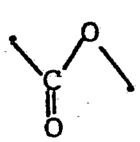 (depsipeptides)

Ondetti et al., Chemistry and Biology of Peptides, ed. J. Meienhofer, Ann. Arbor Science pp. 525-531, 1972.

B. 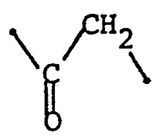 (ketomethylene)

(1) Natarajan et al., Peptides. Synthesis-Structure-Function, ed. D. H. Rich and E. Gross, Pierce Chem. Co., Rockford, Ill., pp. 429-433, 1981.

(2) Van Lommen et al., European Peptide Symposium 16th, Peptides 1980, ed. K. Brunfeldt, Scriptor, Copenhagen, pp. 248-252, 1981.

(3) Almquist et al., J. Med. Chem., 23:1392-1398, 1980.

C. 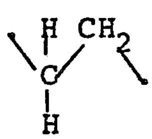 (ethylene)

Kawasaki and Maeda, Biochem. Biophys. Res. Comm. 106:113-116, 1982.

D. 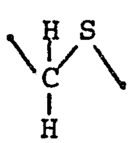 (methylenethio)

(1) Natarajan et al., Id.

(2) Fok and Yankellov, Biochem. Biophys. Res. Comm. 74: 273-278, 1977.

(3) Spatola et al., Peptides. Structure-Function-Biological Function, ed. E. Gross and J. Meienhofer, Pierce Chem. Co., Rockford, Ill., pp. 273-276, 1979.

(4) Spatola and Bettag, J. Org. Chem. 46: 2393-2394, 1981.

E. 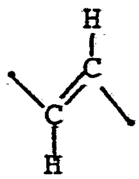 (ethylene)

(1) Natarajan et al., Id.

(2) Hann et al., J. Chem. Soc. Chem. Comm., 234-235, 1980.

(3) Cox et al., J. Chem. Soc. Chem. Comm., 799-800, 1980.

F. 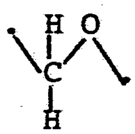 (methylene ether)

Ondetti et al., Id.

G. 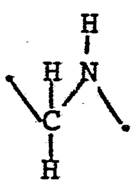 (methylene aza, or reduced isostere)

(1) Van Lommen et al., Id.

(2) Atherton et al., J. Chem. Soc. (C), 3393-3396, 1971.

(3) Parry et al., <u>Chemistry and Biology of Peptides</u>, ed. J. Meienhofer, Ann Arbor Science, pp. 541-544, 1972.

(4) Hudson et al., <u>Int. J. Peptide Protein Res.</u> 15: 122-129, 1979.

(5) Frank and Desiderio, <u>Anal. Biochem</u>. 90: 413-419, 1978.

<u>H</u>. 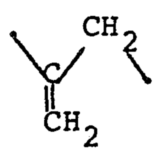 (exomethylene)

Prepared from ketomethylene by Wittig reaction.

<u>I</u>. 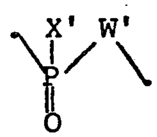

(1) Jacobson and Bartlett, <u>JACS</u> 103: 654-657, 1981.

(2) Jennings-White and Almquist, <u>Tet. Lett</u>., 23: 2533-2534, 1982.

(3) Morton et al., <u>Tet. Lett</u>., 23: 4123-4126, 1982.

For example, the compound:

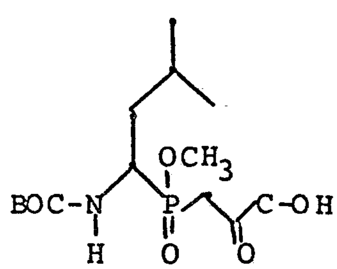

can be prepared in accordance with the following scheme:

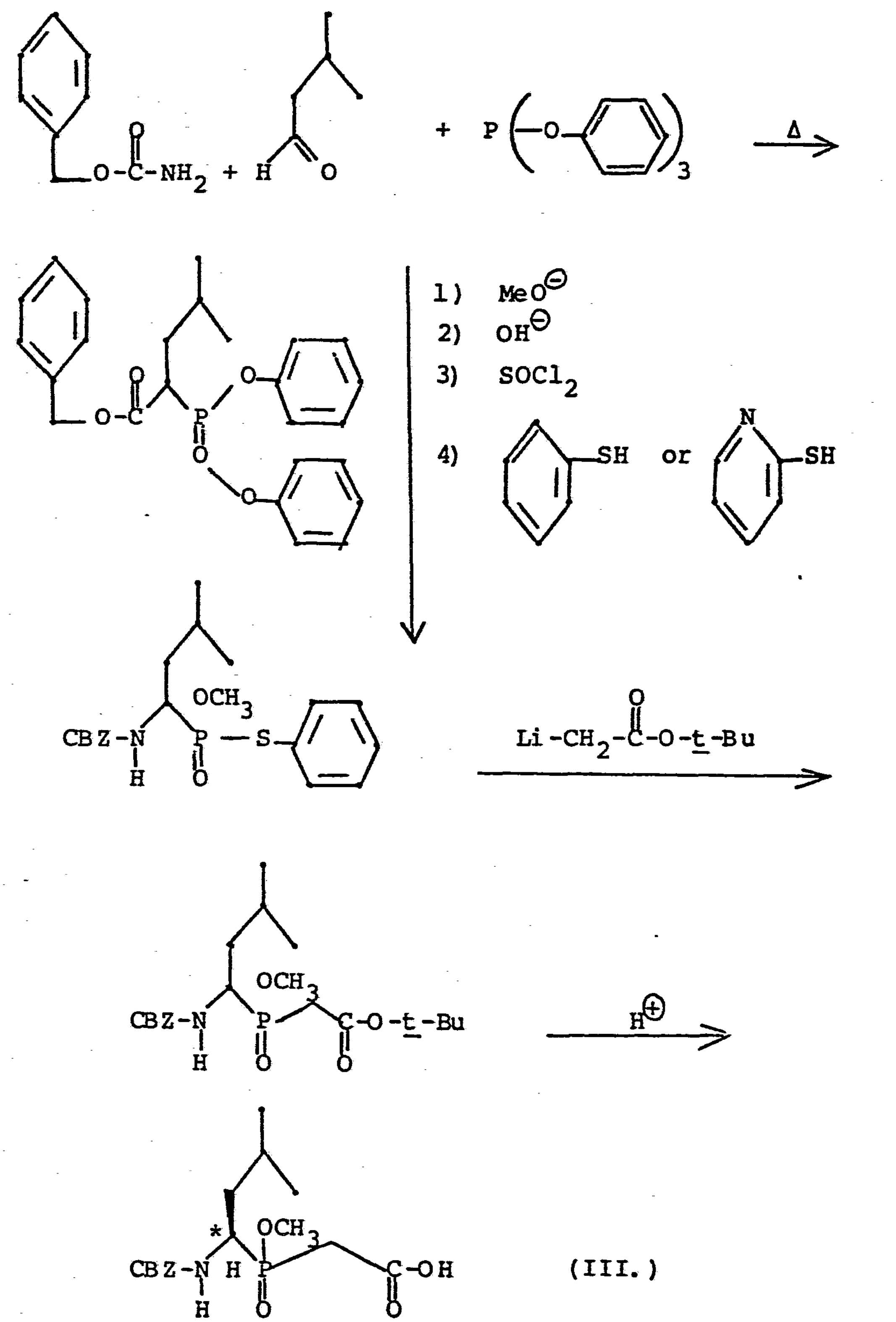


(III.)

(mixture of two pairs of diastereomers; two isomers at *; active isomer indicated).

which can be incorporated into the synthesis for the overall peptide of the present invention, or converted to the α-BOC derivative by hydrogenation over Pd/C catalyst, followed by treatment with $(BOC)_2O$. Incorporation of (III.) or its BOC analog into a peptide sequence gives, after alkaline hydrolysis of the phosphinate ester, the free phosphinate ( 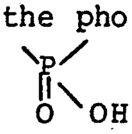 ). The product will contain two isomers at *; the active diastereomer has the relative configuration as an L amino acid, i.e., R-isomer in this case.

Also, the compound: 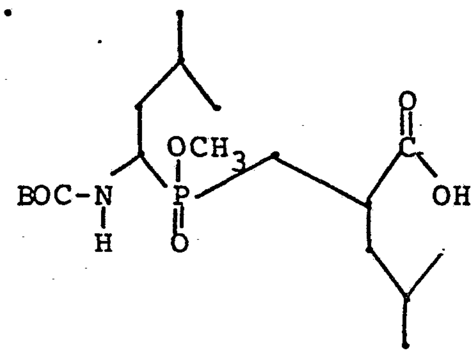

can be prepared in a fashion analgous to that described for (III.) above, for example from:

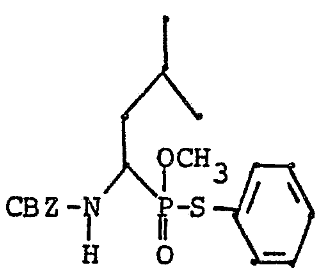

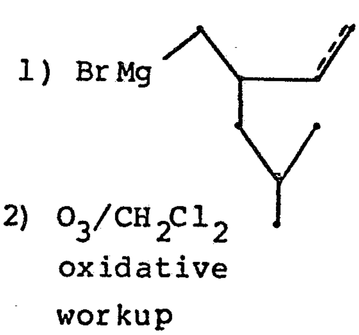

3) Jones oxidation

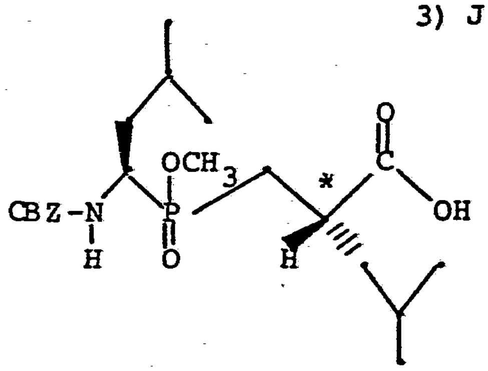

(IV.)

(active isomer shown; other isomers obtained as well)

Incorporation of (IV.) or its N-BOC analog proceeds as for (III.) above, with removal of the methyl phosphinate ester by hydrolysis (alkaline) to give the free phosphinate. The active isomer shown at * has the side chains in the relative configuration of the dipeptide that they mimic. For synthesis of

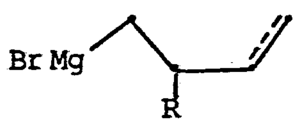

, see Jennings-White and Almquist, <u>Id</u>.

<u>J.</u>

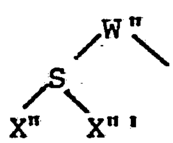

Morton <u>et</u> <u>al</u>., <u>Id</u>.

For example, the compound:

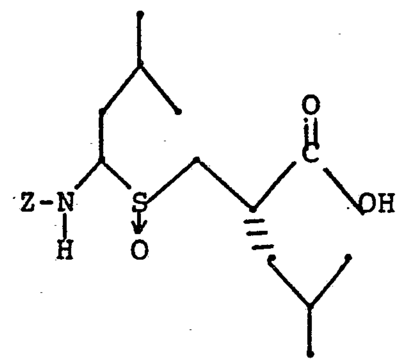

can be prepared in accordance with the following scheme:

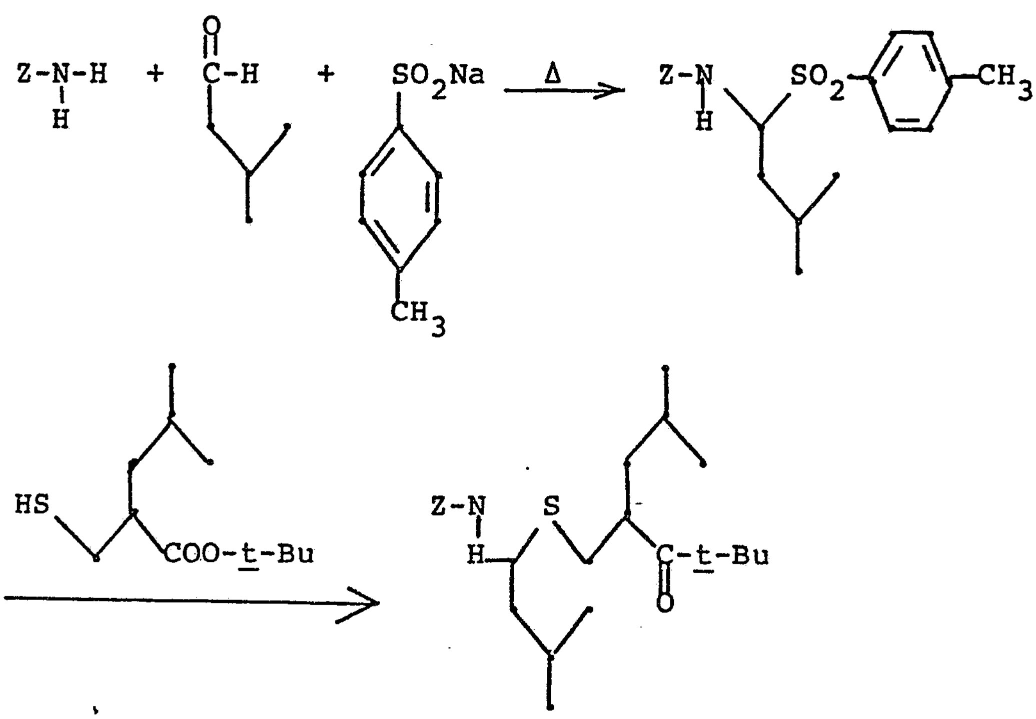

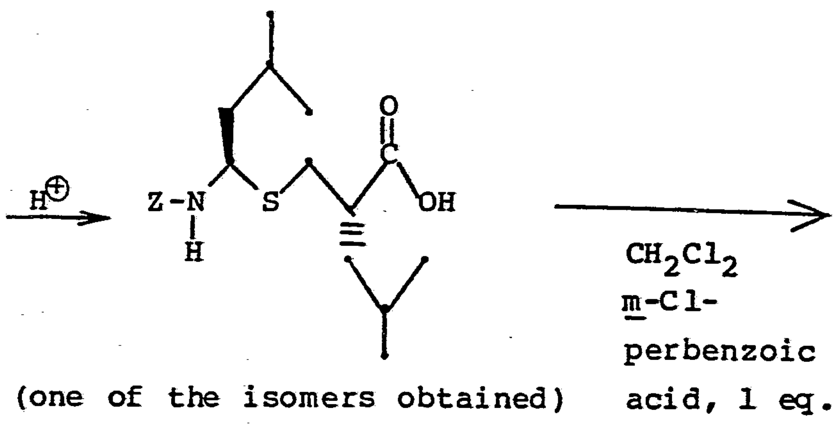


(one of the isomers obtained)

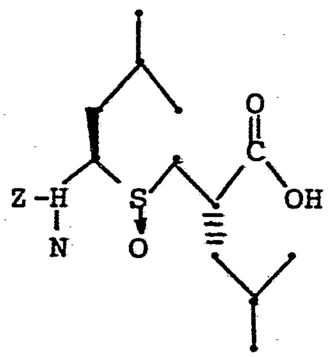

The sulfone ( 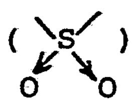 ) can be obtained using excess m-Cl-perbenzoic acid.

Use of 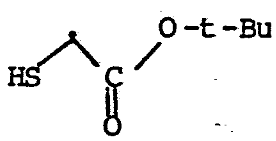 in the second step gives as the final product:

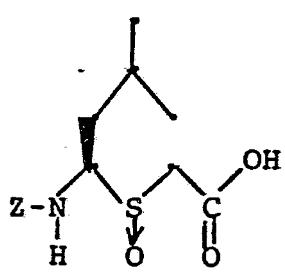

K. 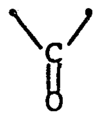 and 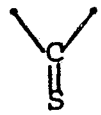

Prepared from the alcohol; see Rich et al., Biochem. Biophys. Res. Comm. 104:1127-1133, 1982.

Conversion of the ketone to the thioketone is with use of:

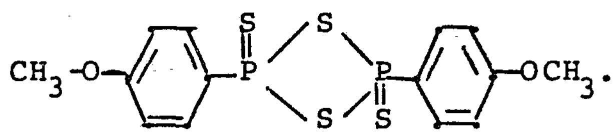

L. 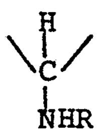

Obtained from 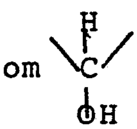 in accordance with the scheme outlined below; the R substituents are attached by conventional methods to the free amine (R=H):

Synthesis of protected 3-amino-3-deoxy-(3S,4S)-Statine:

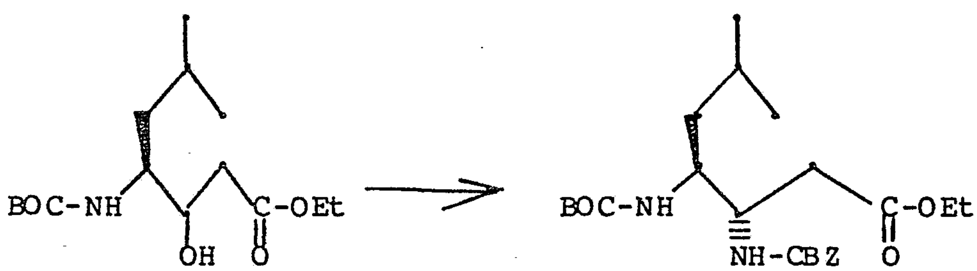

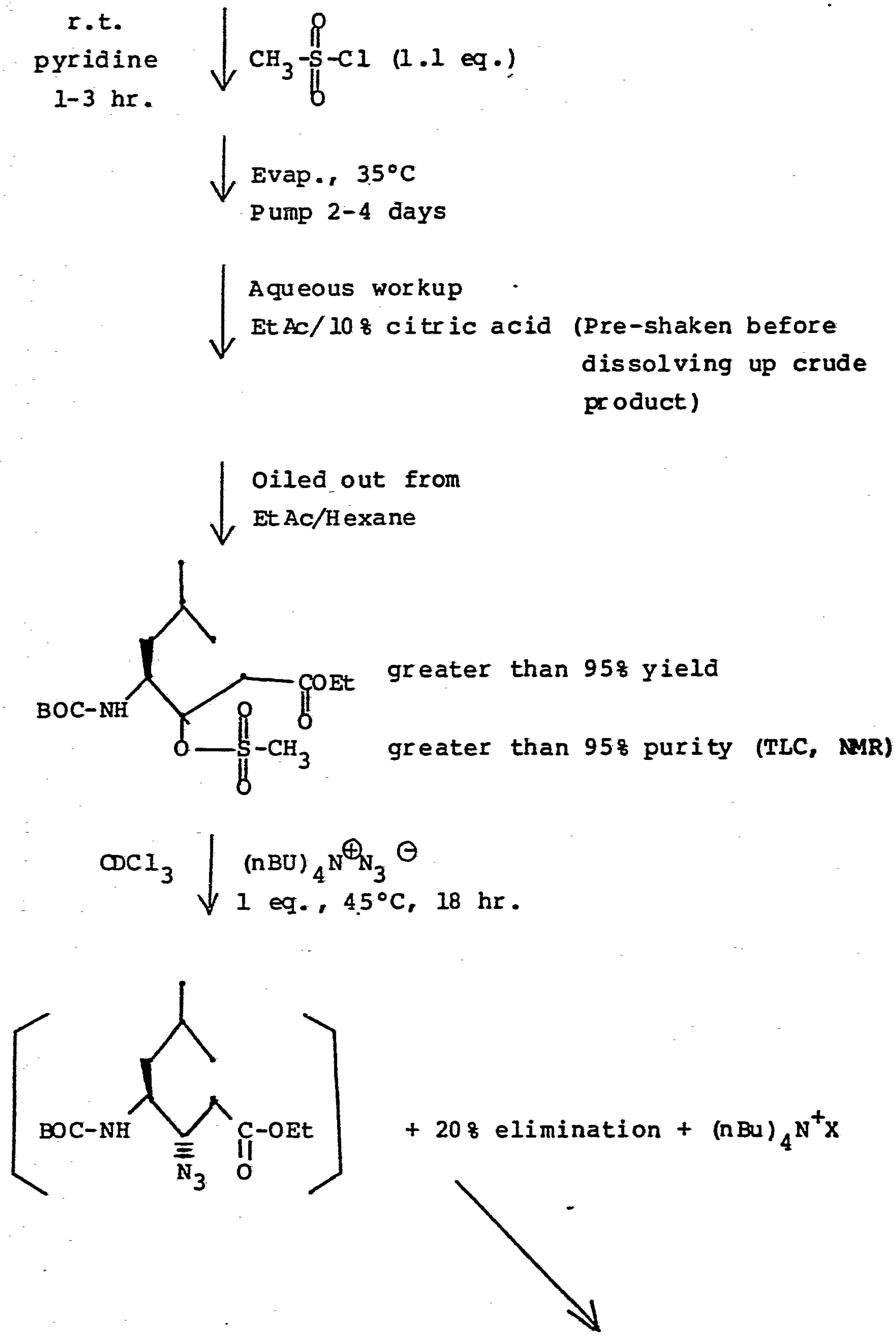
r.t.
pyridine
1-3 hr.
(1.1 eq.)
Evap., 35°C
Pump 2-4 days
Aqueous workup
EtAc/10% citric acid (Pre-shaken before dissolving up crude product)
Oiled out from
EtAc/Hexane
greater than 95% yield
greater than 95% purity (TLC, NMR)
CDCl3
1 eq., 45°C, 18 hr.
+ 20% elimination

Aqueous workup

100% elimination

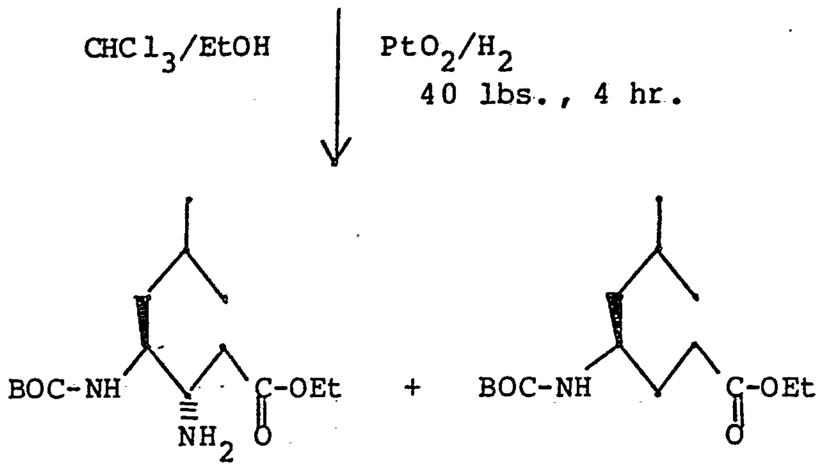


isolated by extraction into weak acid.

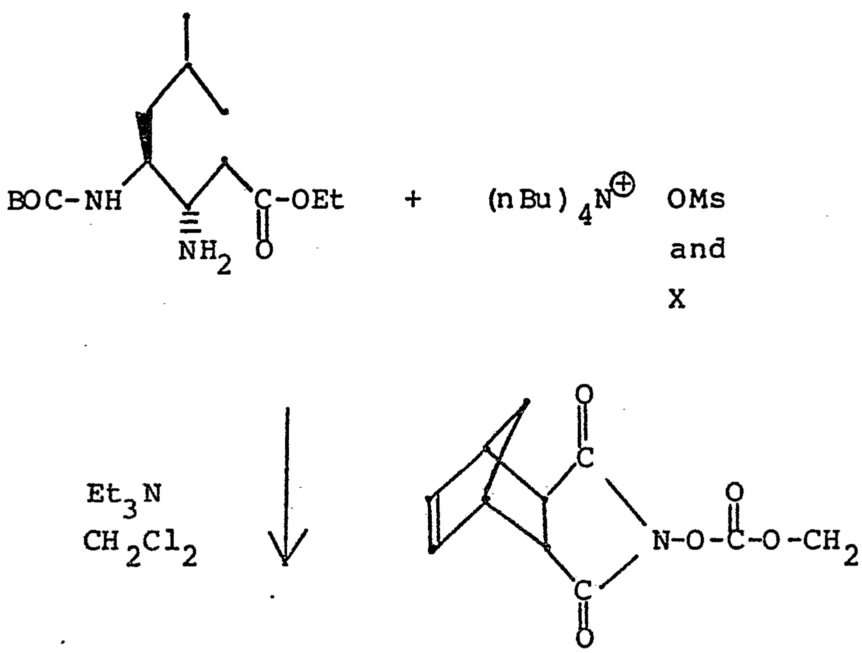

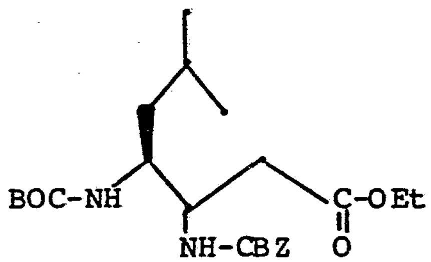
+ excess CBZ reagent

predominantly at
as shown

Base hydrolysis gives the free acid for incorporation into the synthesis of the overall peptides of the present invention.

M.

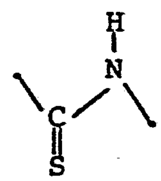

Obtained from the amide according to the method described by Clausen et al., Seventh American Peptide Symposium, 1981:

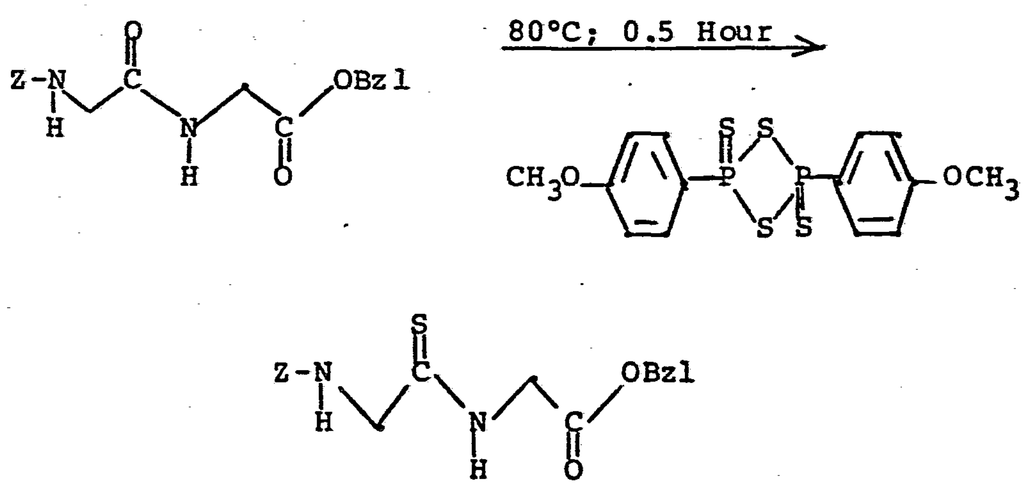

N. 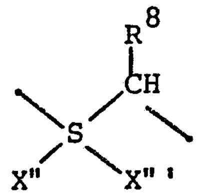

(1) Natarajan et al., Id.

(2) Fok and Yankellov, Id.

(3) Spatola et al., Id.

(4) Spatola and Bettag, Id.

(5) Spatola et al, Proceedings of the Seventh American Peptide Symposium, ed. E. Gross and D. H. Rich, pp. 613-616, 1981.

II. Where the Component G Is a 2-Substituted Statine: an efficient method of preparing the 2-substituted statine component G in the required 2R,3S,4S configuration begins with the preparation of protected phenylalanine aldehyde 1 in three steps beginning from phenylalanine, illustrated as follows:

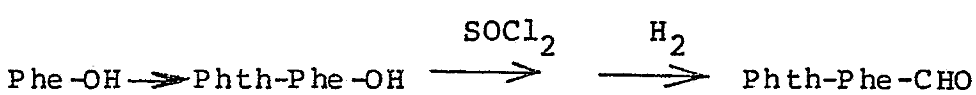


1

This aldehyde 1 can then be reacted with the ketone silylacetal 2 in a titanium mediated aldol condensation to afford an approximately 1:1 mixture of 3a and 3b, illustrated as follows:

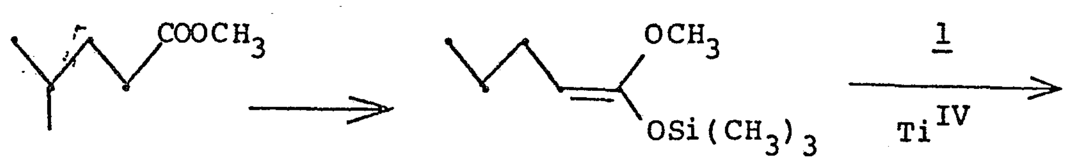


2

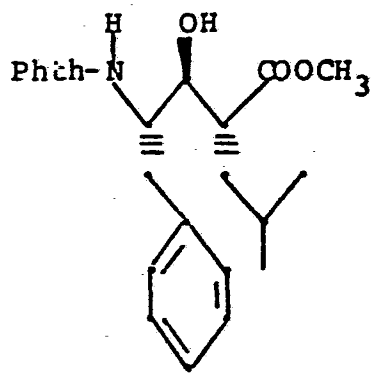

+ isomer

2R,3S,4S 2S,3R,4S

3a 3b

Diasterioselectivity favors by 95% formation of the 3a isomer, and the two diastereomers are thus readily separated by chromatography.

The configurations of the chiral centers can be established as follows: treatment of the phthalimido methyl esters 3a and 3b with excess hydrazine gives the respective amino acyl hydrazides 4a and 4b, which are then converted in a two step/one pot procedure to the corresponding lactams 5a and 5b, to which stereochemical assignments can be made based on PMR analysis. These reactions may be illustrated as follows:

3a
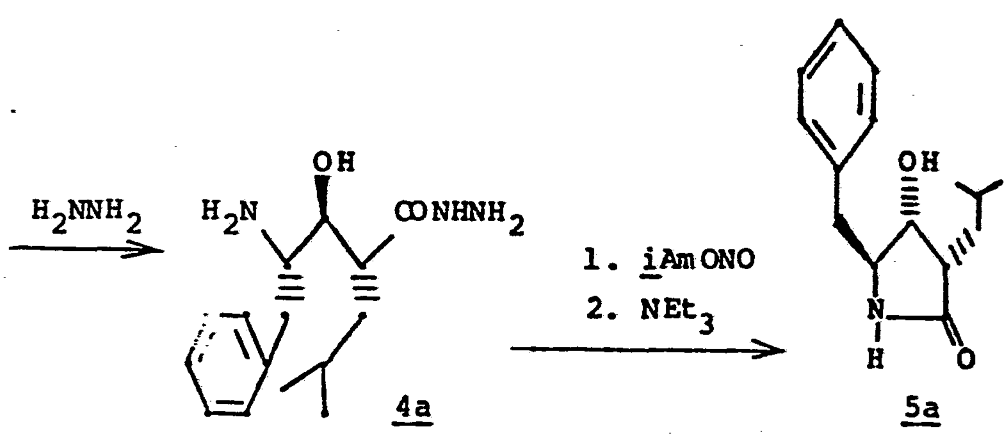


4a 5a

3b

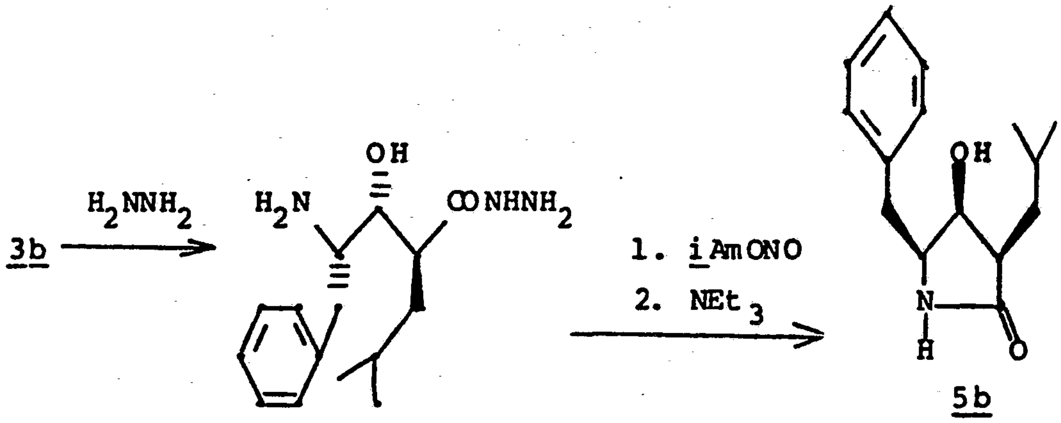


5b

Alternatively, the benzyl ester 6, rather than the methyl ester, may be used to form the ketone silylacetal 7, which can then be reacted with phthalyl phenylalanine aldehyde and phthalyl leucine aldehyde, for example, to give 8a and 8b, illustrated as follows:

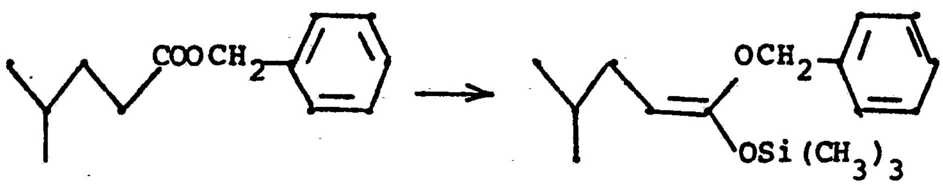

6 7

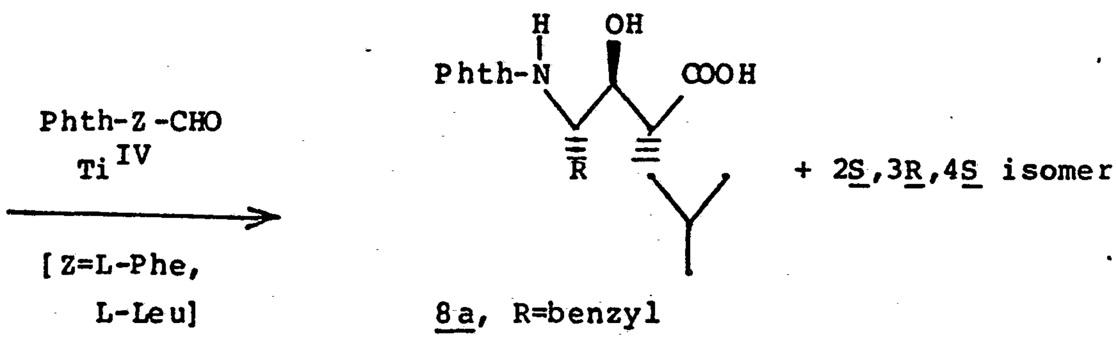


+ 2S,3R,4S isomer

8a, R=benzyl
8b, R=iso-butyl

Separation of the isomers followed by hydrogenation gives a protected 2-substituted statine component which can be used to prepare peptides of Formula I in accordance with well-known methods of peptide synthesis.

Preparation of a renin inhibitory peptide of the present invention containing (2-*i*-Bu)-Sta may be schematically represented as follows:

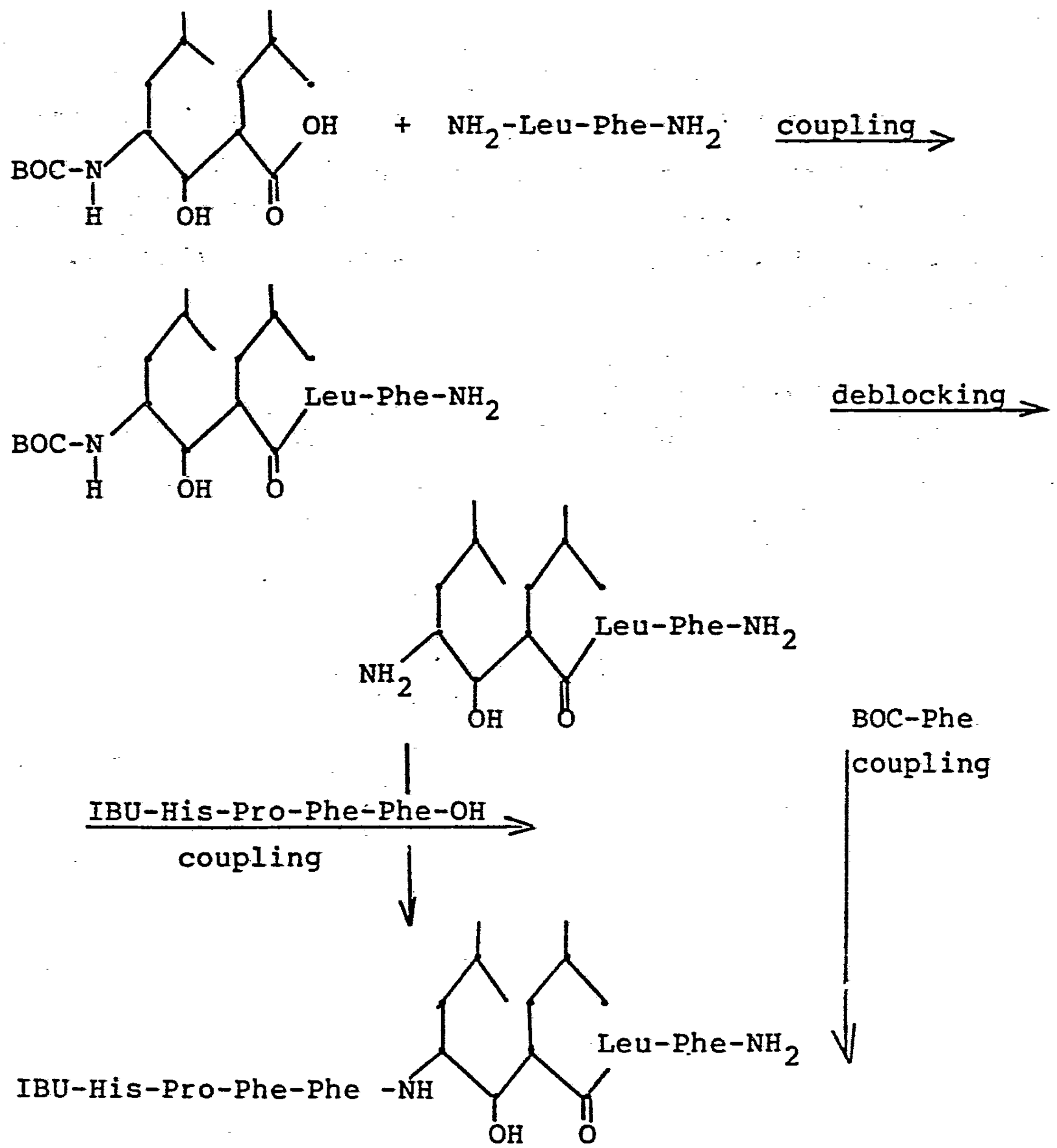

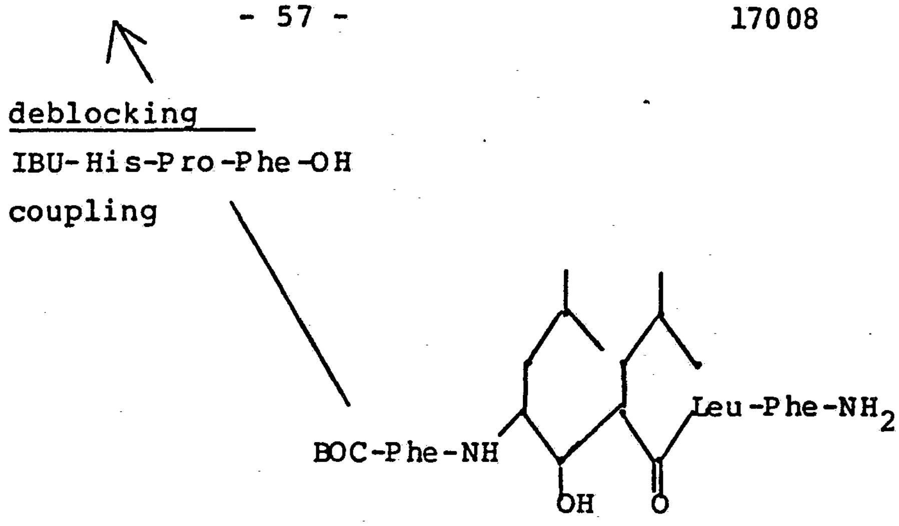


III. <u>Where the Component G Is the Amino Acid Statine Itself:</u> it may be prepared in accordance with the procedure described by Rich et al., J. Org. Chem. 43: 3624, 1978.

The phenyl analog of statine (3S,4S)-4-amino-3-hydroxy-5-phenylpentanoic acid (AHPPA) can be prepared in accordance with the procedure described by Rich et al., J. Med. Chem. 23: 27-33 (1980).

The cyclohexylalanine analog of statine, (3S,4S)-4-amino-5-cyclohexyl-3-hydroxypentanoic acid (ACHPA) can be prepared by catalytic hydrogenation (using $H_2$/Rh on alumina, or other sutiable catalyst) of the BOC-AHPPA, prepared as described in the paragraph immediately above. Alternatively, this and similar statine analogs can be prepared in accordance with the procedure described for statine, where the BOC-Leu starting material is replaced with the amino acid containing the desired side chain. Thus, BOC-ACHPA can also be prepared starting from BOC-L-cyclohexylalanine, itself prepared, for example, by catalytic reduction of BOC-Phe, in the same manner as described for BOC-AHPPA.

The novel inhibitory peptides of the present invention are prepared by using the solid phase sequential synthesis technique.

In the following description several abbreviated designations are used for the amino acid components, certain preferred protecting groups, reagents and solvents. The meanings of such abbreviated designations are given below in Table I.

TABLE I

| Abbreviated Designation | Amino Acid |
|---|---|
| Ala | L-alanine |
| Arg | L-arginine |
| Gly | L-glycine |
| His | D or L-histidine |
| Ile | L-isoleucine |
| Leu | L-leucine |
| Lys | L-lysine |
| Met | L-methionine |
| Orn | L-ornithine |
| Phe | L-phenylalanine |
| Ser | L-serine |
| Sar (N-methylglycine) | L-sarcosine |
| Thr | L-threonine |
| Trp | L-tryptophan |
| Tyr | L-tyrosine |
| Val | L-valine |

| Abbreviated Designation | Protecting Groups |
|---|---|
| BOC | tert-butyloxycarbonyl |
| CBZ | benzyloxycarbonyl |
| DNP | dinitrophenyl |
| OMe | methyl ester |

| Abbreviated Designation | Activating Groups |
|---|---|
| HBT | 1-hydroxybenzotriazole |

| Abbreviated Designation | Condensing Agents |
|---|---|
| DCCI | dicyclohexylcarbodiimide |
| DPPA | diphenylphosphorylazide |

| Abbreviated Designation | Reagents |
|---|---|
| TEA | triethylamine |
| TFA | trifluoroacetic acid |

| Abbreviated Designation | Solvents |
|---|---|
| A | ammonium hydroxide (conc.) |
| AcOH | acetic acid |
| C | chloroform |
| DMF | dimethylformamide |
| E | ethyl acetate |

| Abbreviated Designation | Solvents |
|---|---|
| M | methanol |
| P | pyridine |
| THF | tetrahydrofuran |
| W | water |

The synthesis of the peptides of the present invention by the solid phase technique is conducted in a stepwise manner on chloromethylated resin. The resin is composed of fine beads (20-70 microns in diameter) of a synthetic resin prepared by copolymerization of styrene with 1-2 percent divinylbenzene. The benzene rings in the resin are chloromethylated in a Friedel-Crafts reaction with chloromethyl methyl ether and stannic chloride. The Friedel-Crafts reaction is continued until the resin contains 0.5 to 5 mmoles of chlorine per gram of resin.

The amino acid selected to be the C-terminal amino acid of the linear peptide is converted to its amino protected derivative. The carboxyl group of the selected C-terminal amino acid is bound covalently to the insoluble polymeric resin support, as for example, as the carboxylic ester of the resin-bonded benzyl chloride present in chloromethyl-substituted polystyrene-divinylbenzene resin. After the amino protecting group is removed, the amino protected derivative of the next amino acid in the sequence is added along with a coupling agent, such as dicyclohexylcarbodiimide. The amino acid reactant may be employed in the form of a carboxyl-activated amino acid such as ONP ester, an amino acid azide, and the

like. Deprotection and addition of successive amino acids is performed until the desired linear peptide is formed.

The selection of protecting groups is, in part, dictated by particular coupling conditions, in part by the amino acid and peptide components involved in the reaction.

Amino-protecting groups ordinarily employed include those which are well known in the art, for example, urethane protecting substituents such as benzyloxy-carbonyl (carbobenzoxy), p-methoxycarbobenzoxy, p-nitrocarbobenzoxy, t-butyoxycarbonyl, and the like. It is preferred to utilize t-butyloxycarbonyl (BOC) for protecting the α-amino group in the amino acids undergoing reaction at the carboxyl end of said amino acid. The BOC protecting group is readily removed following such coupling reaction and prior to the subsequent step by the relatively mild action of acids (i.e. trifluoroacetic acid, or hydrogen chloride in ethyl acetate).

The OH group of Thr and Ser can be protected by the Bzl group and the -amino group of Lys can be protected by the INOC group or the 2-chlorobenzyloxycarbonyl (2-Cl-CBZ) group. Neither group is affected by TFA, used for removing BOC protecting groups. After the peptide is formed, the protective groups, such as 2-Cl-CBZ and Bzl, can be removed by treatment with HF or by catalytic hydrogenation.

After the peptide has been formed on the solid phase resin, it may be removed from the resin by a variety of methods which are well known in the art. For example the peptide may be cleaved from the resin with hydrazine, by ammonia in methanol, or by methanol plus a suitable base.

Preparation of the novel inhibitory peptides of the present invention utilizing the solid phase technique is illustrated in the following examples, which however, are not intended to be any limitation of the present invention.

EXAMPLE 1

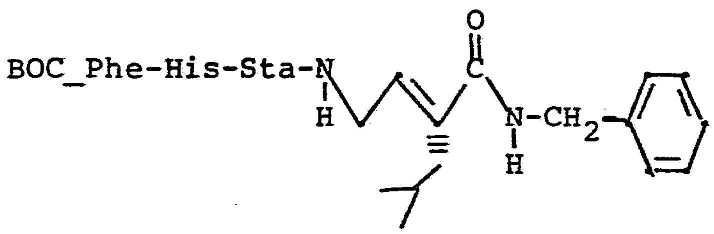

Step A.

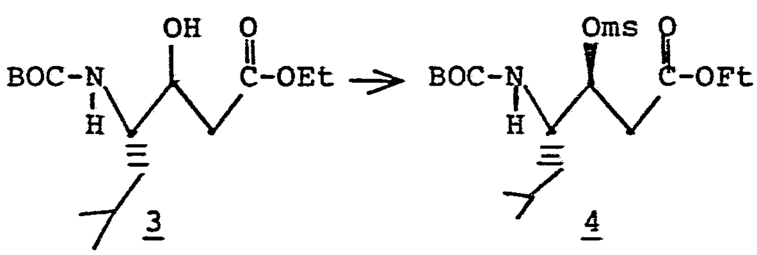

3 4

To an ice cold, stirred solution of BOC-statine ethyl ester 3 (2.60 g, 8.57 mmole) in 10 ml of pyridine is added via syringe 0.66 ml (8.57 mmole) of trifluoromethane sulfonyl chloride. Within minutes, pyridinium hydrochloride precipitates from solution. The reaction mixture is protected from moisture and allowed to stand at room temperature overnight. The reaction mixture is filtered and the filtrate concentrated to give a light orange oil.

The crude product is filtered through 5-10 g of silica gel (ether elution) to give 3.0 g of a pale yellow oil which is used without further purification. Compound 4 is prone to hydrolysis to 3 and must be used without delay.

Step B.

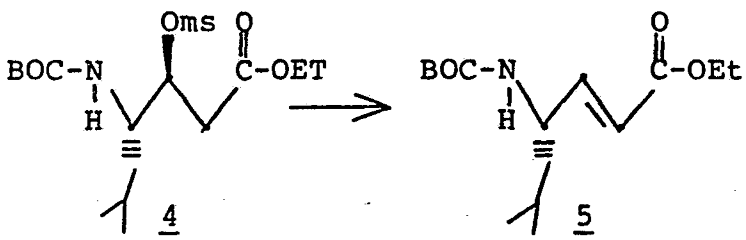

1,5-Diazabicyclo[4.3.0]non-5-ene (DBN) (0.94 ml, 7.54 mmole) is added in one portion to a stirred solution of 5 (2.9 g, 6.85 mmole) in 25 ml of dry tetrahydrofuran. The reaction is slightly exothermic and within minutes, a thick white precipitate is formed. The reaction mixture is allowed to stir for several hours more and is then filtered. The filtrate is concentrated *in vacuo*. The residual oil is partitioned between ether and 10% citric acid solution. The organic phase is washed with citric acid (2 x 40 ml) and brine, then dried ($Na_2SO_4$) and concentrated. Flash chromatography on silica gel (7:3 hexane-ethyl acetate elution) provides the analytical sample as an oil (1.6 g). ir; pmr($CDCl_3$): olefinic protons 5.9 (d, J=17), 6.83 (d x d, J=17 and 5). J (coupling constant) is consistent with *trans* double bond.

Step C.

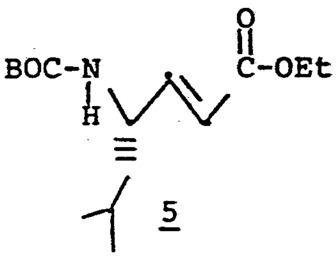

5

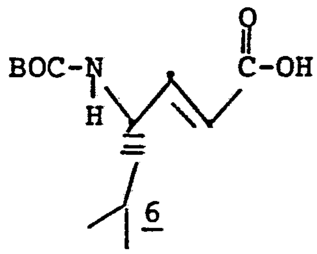

6

The α,β-unsaturataed ester 5 (1.5 g, 5.3 mmole) is dissolved in water/dioxane 20 ml (1:1 v/v) and treated with 7 ml (1.3 equivalents) of 1M sodium hydroxide solution. After 3 hours, 1 ml of 1M sodium hydroxide solution is again added. Dioxane is removed *in vacuo* after a total of 4 hours reaction time. The alkaline aqueous residue is diluted to 25 ml with water and washed with ether (2 x 25 ml). The aqueous phase is acidified with 10% citric acid and extracted with ether and chloroform. The combined organic extracts are washed with brine and dried ($Na_2SO_4$). Concentration under reduced pressure affords 1.46 g of 6.

Step D.

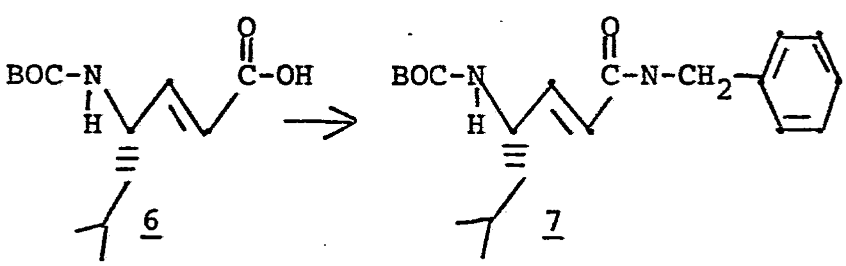

6 7

The acid 6 (400 mg, 1.6 mmole) is dissolved in 4 ml of methylene chloride under nitrogen. N-methylmorpholine (0.18 ml, 1.6 mmole) is added and the solution is cooled to -5°C. Isobutylchloroformate (0.21 ml, 1.6 mmole) is added and after 15 minutes, 0.22 ml (1.92 mmole) of benzylamine is added to the reaction mixture. After 30 minutes at -5°C the reaction mixture is warmed to room temperature, stirred 1 hour more and diluted with 70 ml of methylene chloride. The organic phase is washed in succession with 10% citric acid (2 x 30 ml), saturated sodium bicarbonate solution (2 x 30 ml), and brine. The organic extracts are dried ($Na_2SO_4$) and concentrated to yield 460 mg of a white solid identified as 7.

Step E.

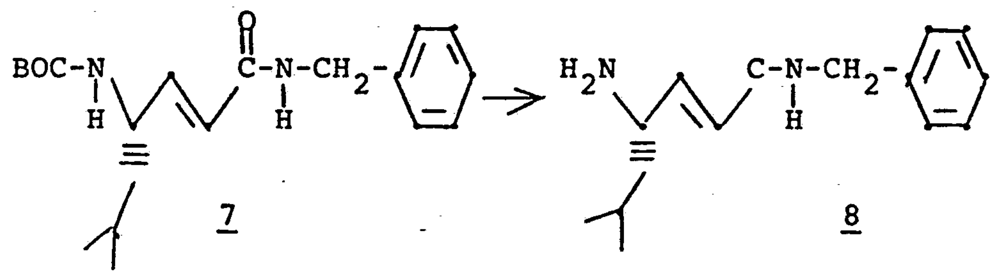

7 8

The BOC-amide 7 (240 mg, 0.69 mmole) is dissolved in 20 ml of ethyl acetate, cooled to 0°C and treated with a stream of hydrogen chloride gas for 1 hour. Solvent and excess reagent are removed under reduced pressure to afford 200 mg of a pale yellow solid.

Step F.

BOC-Phe-His-Sta-OEt → BOC-Phe-His-Sta-$NHNH_2$

1 2

The tripeptide ester BOC Phe-His-Sta-OEt 1 (520 mg, 0.89 mmole) is dissolved in 4 ml of methanol and treated with 2 ml (62 mmole) of 95% hydrazine. After 10 minutes at room temperature the reation mixture is concentrated *in vacuo* (0.1 Torr) to afford 420 mg of 2 as a tan powder.

Step G.

BOC-Phe-His-Sta-$NHNH_2$ +

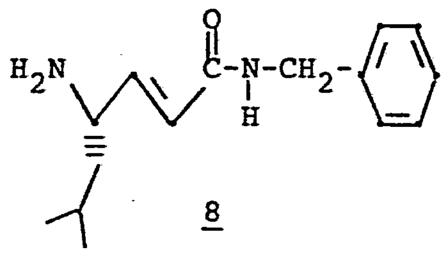

2 8

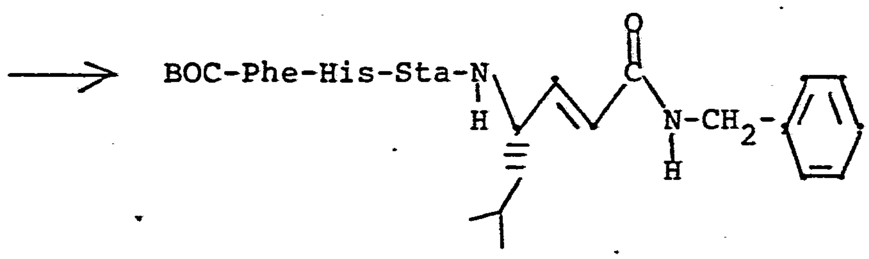

The hydrazide 2 (240 mg, 0.42 mmole) is dissolved in 2 ml of dry dimethylformamide under nitrogen. The solution is cooled to -20°C and the pH of the reaction mixture adjusted to approximately 0.5-1.0 with tetrahydrofuran saturated with hydrogen chloride. Isoamyl nitrite is then added in 50 µl increments at 15-20 minute intervals until a positive potassium iodide-starch test is obtained (250 µl total). The amine salt 8 (190 mg, 0.67 mmole) is dissolved in 2 ml of dimethylformamide and added to the reaction mixture. After addition is complete, the pH of the reaction mixture is adjusted to

7.5-8.0 with triethylamine and the reaction mixture is allowed to stir at -20°C for 20 hours. The reaction is filtered and the filtrate concentrated. The resulting residue is partitioned between ethyl acetate and water. The organic phase is washed in succession with 10% citric acid solution (2 x 50 ml), 50% sodium bicarbonate solution (2 x 50 ml), and brine. The organic extracts are dried ($Na_2SO_4$) and rotoevaporated to yield 140 mg of a yellow semi-solid. Chromatography on silica gel (80:10:1 $CHCl_3$-ethanol-ammonia elution) affords the title compound as a pale yellow solid.

EXAMPLE 2

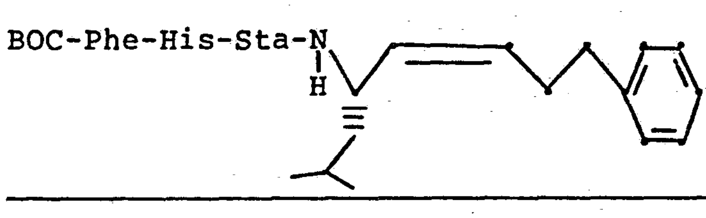

Step A.

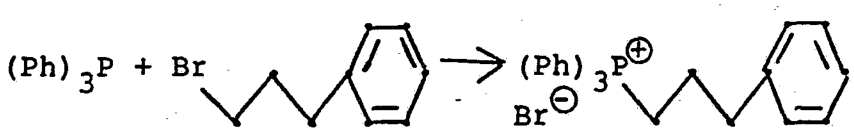

2 3

3-Phenyl-1-bromopropane (19 ml, 72.8 mmole) and triphenyl phosphine (19.11 g, 72.8 mmole) are combined at room temperature and immersed in a preheated oil bath at 150°C. Heating is continued for 1.5 hours at 150-160°C. The dark, brown solution is cooled and diluted with 200 ml of acetone. The

acetone is decanted and the residue is triturated with hot ethyl acetate. In this way, an off-white solid is obtained which on further washing with ethyl acetate gives 24.2 g of 3 as a white powder, m.p. 203-207°C.

Step B.

BOC-Leu-CHO + 3 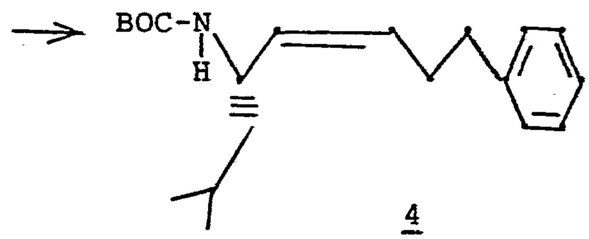

1 4

A rapidly stirred suspension of 3 (1.82 g, 3.95 mmole) in 15 ml of dry tetrahydrofuran is treated dropwise under nitrogen at 0°C with n-butyl lithium (1.4N, 2.82 ml, 3.95 mmole). The reaction mixture, which becomes homogeneous and colors to dark brown, is cooled to -78°C and treated with 5 ml of tetrahydrofuran containing 0.5 g (2.32 mmole) of BOC-leucine aldehyde 1. After four hours at -78°C the reaction mixture is warmed to -10°C for 1 hour and then quenched with saturated ammonium chloride solution. The reaction is partitioned between ether and brine. The organic phase is then washed with 10% citric acid solution (3 x 20 ml), 50% sodium bicarbonate solution (3 x 20 ml), and brine. Rotoevaporation of the dried ($Na_2SO_4$) extracts affords 0.42 g of crude product as a yellow oil. The analytical sample is obtained by chromatography of the crude product on silica gel (hexane-ethyl acetate 9:1 elution).

Step C.

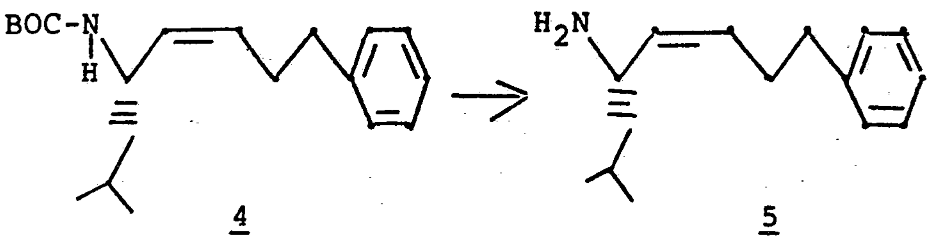

4 5

The BOC-olefin 4 (410 mg, 1.29 mmole) is dissolved in 20 ml of ethyl acetate, cooled to 0°C, and treated with hydrogen chloride gas for 1 hour. Concentration of the reaction mixture *in vacuo* and then under high vacuum gives the HCl salt 5 as a beige solid (310 mg).

Step D.

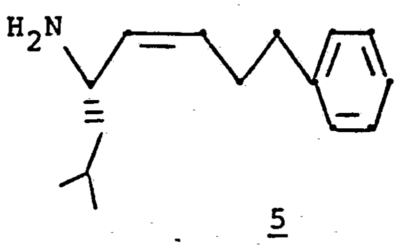 ·HCl + BOC-Phe-His-Sta-$NHNH_2$

5 6

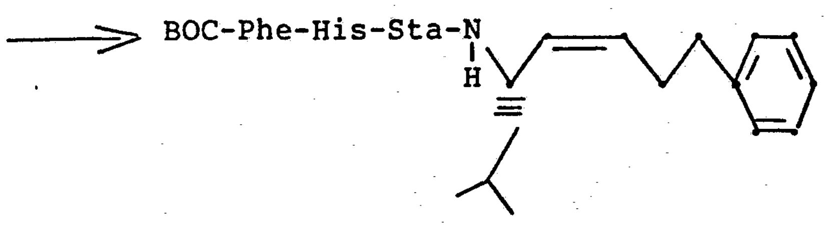

BOC Phe-His-Sta-$NHNH_2$ (240 mg, 0.42 mmole) [obtained as described in Example 1, Step F] is converted to the corresponding BOC-Phe-His-Sta-$N_3$ (azide) with isoamylnitrite (200 µl) [using identical reaction conditions as described in Example 1, Step G]. The amine salt 5 (160 mg, 0.63 mmole) is

then added [and the reaction is carried out and worked-up as described in Example 1, Step G]. The analytical sample (70 mg) is obtained after silica gel chromatography (80:10:1 $CHCl_3$-ethanol-ammonia elution) as a pale yellow solid.

EXAMPLE 3

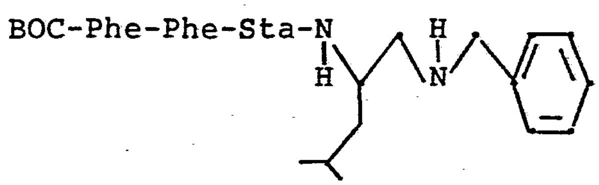

Step A.

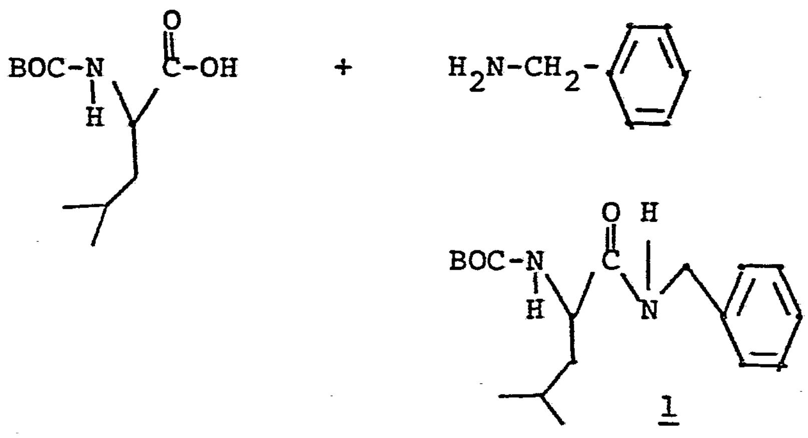

1

N-Methylmorpholine (9.14 ml, 83.2 mmole), and BOC-leucine hydrate (20.0 g, 83.2 mmole) are dissolved in $CH_2Cl_2$ (200 ml) and the solution cooled to -5°C. Isobutylchloroformate (10.8 ml, 83.2 mmole) is added and the solution stirred 15 min. Benzylamine (10.9 ml, 99.8 mmole) is added and the solution stirred 15 min. The solution is warmed to 25°C ( 30 min.) and dichloromethane (300 ml) added. The organic layer is washed with 10% citric acid (2 x 150 ml), water (1 x 150 ml), 10% sodium bocarbonate (2 x 150 ml), and brine (2 x 150 ml);

dried over $Na_2SO_4$; and filtered. The filtrate is evaporataed under reduced pressure and the residue dried at 25°C in a vacuum oven to give 24.75 g (93% yield) of 1 as a waxy colorless solid.

Step B.

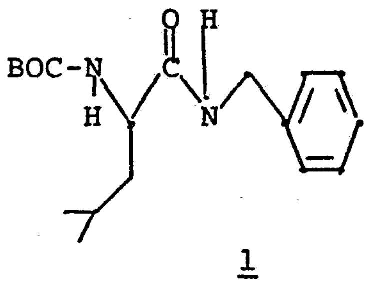

1

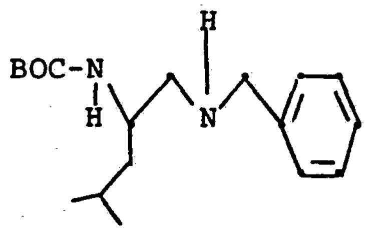

2

Compound 1 from Step A. (1.0 g, 3.1 mmole) is dissolved in tetrahydrofuran (6.25 ml) and the solution cooled to -25°C. Diborane (6.25 ml of 1M tetrahydrofuran solution, 6.25 mmole) is added dropwise and the solution stirred 48 hours at -10°C. Methanol (5 ml) is added and the reaction stirred at 25°C for 16 hours. The solvent is removed under reduced pressure and the residue treated with methanol and restripped (3X). Flash chromatography of the final residue using silica gel eluted with 35% ethyl acetate in hexane gives 380 mg (40% yield) of 2 as a light yellow oil.

Step C.

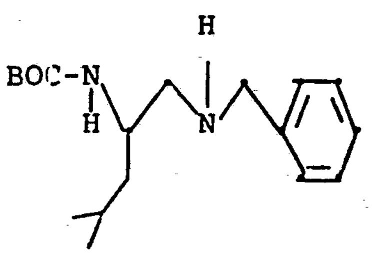

2

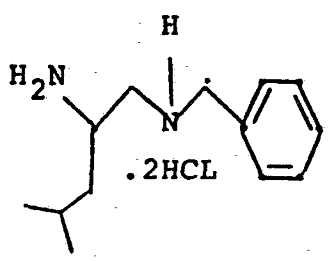

3

Protected amine 2 (540 mg, 1.8 mmol) is dissolved in ethyl acetate (10 ml). The solution is cooled to 0°C, saturatad with HCl (g), and stirred 15 min. The solvent is removed *in vacuo*. The residue is treated with ethyl acetate and restripped (4X) to give a quantitative (490 mg) yield of 3 as an off-white solid.

Step D.

3 + BOC-Phe-Phe-Sta-OH

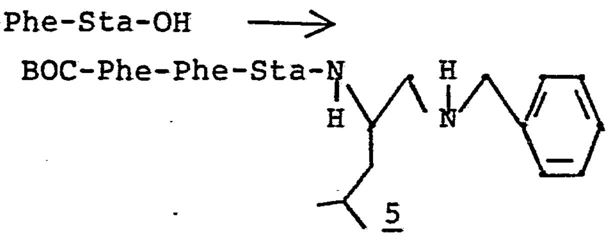

5

BOC-Phe-Phe-Sta-OH (240 mg, 0.421 mmole), diamine dihydrochloride 3 (130 mg, 0.466 mmole), 1-hydroxybenzotriazole hydrate (HBT) (62.9 mg, 0.466 mmole), and 1-ethyl-3-(3-dimethylaminopropyl) carbodiimide hydrochloride (EDC) (895 mg, 0.466 mmole) are dissolved in degassed dimethylformamide (4 ml) under a nitrogen atmosphere. The pH of the solution is adjusted to 9.0-9.5 with triethylamine (0.30 ml, 2.16 mmole). After stirring 24 hours, a second portion of HBT (7 mg, .052 mmole) and EDC (9 mg, .047 mmole) is added and the suspension stirred 6 hours. The dimethylformamide is removed *in vacuo*. The residue is treated with 10% citric acid and extracted with ethyl acetate (3X). The organic layers are combined, washed with $H_2O$ (1X), 10% aqueous sodium bicarbonate (2X), and brine (1X), dried over $MgSO_4$, filtered, and stripped under reduced pressure to give 280 mg of a white foam.

Flash chromatography on silica gel with 150/10/1/1 of dichloromethane/methanol/water/acetic acid gives the desired product 5 (200 mg, 62.7% yield) as a white foam.

## EXAMPLE 4

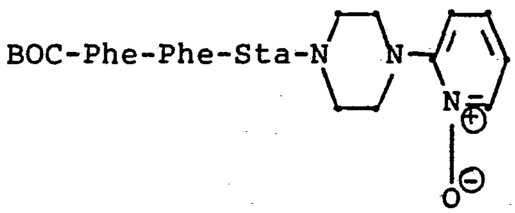

Step A.

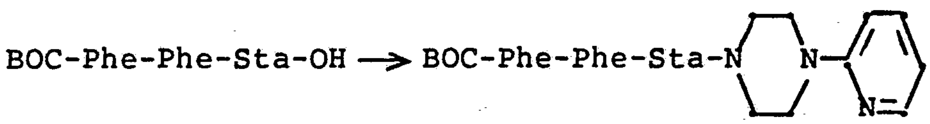

To a solution of 1 ml of dimethylformamide at 0° is added in succession BOC-Phe-Phe-Sta-OH (114 mg, 0.2 mmole), 2-pyridylpiperazine 33.5 µl, 35 mg, 0.22 mmole) diphenylphosphonylazide (47.5 µl, 60.7 mg, 0.22 mmole), and sodium bicarbonate (84 mg, 1 mmole). The resulting suspension is protected from moisture and stirred at 0° for 12 hours. More diphenylphosphonylazide is added (47.5 µl, 0.22 ml) and stirring continued at 0°C. After 2 days the reaction mixture is filtered and the filtrate concentrated *in vacuo*. The residue is chromatographed on silica gel (90:10:1:0.1 chloroform/methanol/water/acetic acid elution) to give 125 mg of the analytical sample as a white solid.

Step B.

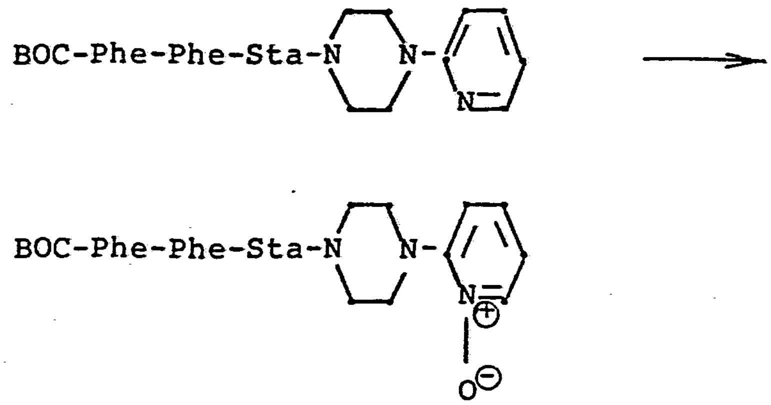

The tri-peptide of Step A. (41 mg, 0.06 mmole) is dissolved in 5 ml of chloroform and the resulting solution is treated with 20 mg of tech. grade m-chloroperbenzoic acid (85%). The reaction mixture is allowed to stand for 19 hours at room temperature and then the solvent is removed under reduced pressure. The residue is chromatographed on silica gel (chloroform-ethanol-ammonia 80:10:1 elution). The material with $R_f$ value of 0.24 is isolated to provide the analytical sample as a white solid (32 mg).

WHAT IS CLAIMED IS:

1. A peptide of the formula:

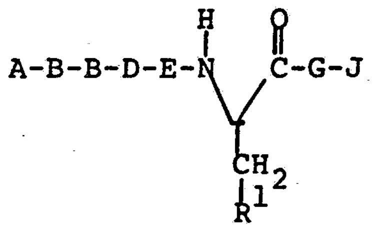

(I.)

wherein:

A is hydrogen; or 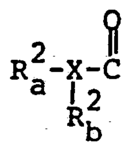

where

X is -O-; -O-CH-; -CH-O-; -CH-; -NH-CH-; or -S-CH-; and

$R_a^2$ and $R_b^2$ may be the same or different and are hydrogen; $W-(CH_2)_n-$ or $W-(CH_2)_m-CH=CH-(CH_2)_p$, where W is hydrogen; $C_{1-4}$alkyl; aryl; $C_{3-7}$cycloalkyl; or $C_{3-7}$cycloalkyl or aryl substituted with up to five members independently selected from the group consisting of $C_{1-8}$alkyl, trifluoromethyl, hydroxy, $C_{1-4}$alkoxy, and halo; n is 0 to 5; m is 0 to 2; and p is 0 to 2; except that where X is -O-, only one of $R_a^2$ or $R_b^2$ is present;

B is absent; glycyl; sarcosyl; or 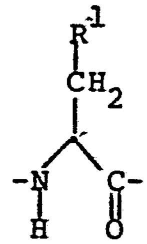, where $R^1$ is as defined further below;

D is absent; or 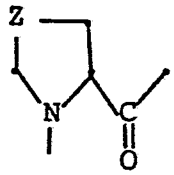, where Z is $-(CH_2)_l-$ and l is 1 or 2; or -S-;

E is absent; or 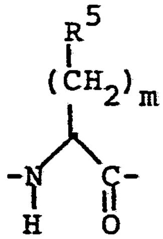, where m is 1 to 4; and $R^5$ is hydrogen; $C_{1-4}$ alkyl; aryl; aryl-$C_{1-4}$ alkyl; aryl $C_{1-4}$ alkyl or aryl where the aryl portion is substituted with up to three members selected from the group consisting of $C_{1-4}$ alkyl, trifluoromethyl, hydroxy, $C_{1-4}$ alkoxy, and halo; or indolyl;

G is (1) 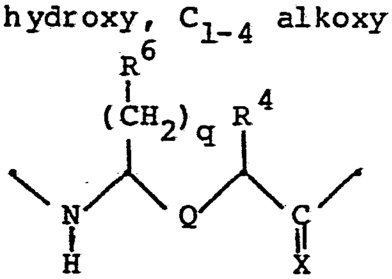 where q is 1 to 4; X is O, or H, H;

$R^4$ is hydrogen; or 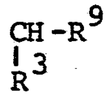, where $R^9$ is hydrogen; $C_{1-4}$alkyl; hydroxy, or $C_{3-7}$cycloalkyl; and

$R^3$ is hydrogen; $C_{1-4}$alkyl; aryl; aryl $C_{1-4}$alkyl; aryl $C_{1-4}$alkyl or aryl substituted with up to three members selected from the group consisting of $C_{1-4}$alkyl, trifluoromethyl, hydroxy, $C_{1-4}$alkoxy, and halo; or indolyl;

$R^6$ is $C_{3-6}$ alkyl; $C_{3-7}$ cycloalkyl; aryl; or $C_{3-7}$cycloalkyl or aryl substituted with up to three members selected from the group consisting of $C_{1-4}$alkyl, trifluoromethyl, hydroxy, $C_{1-4}$alkoxy, and halo; and

Q is 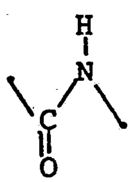 ; 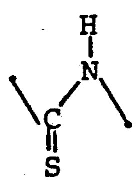 ; 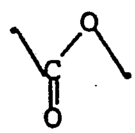 ; 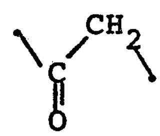 ;

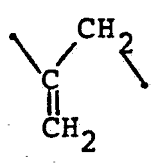 ; 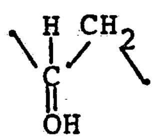 ; 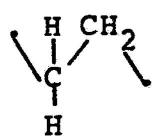 ; 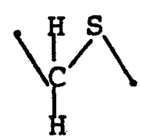 ;

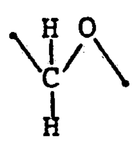 ; 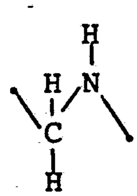 ; 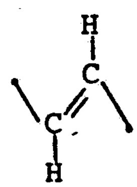 ; 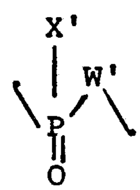 ;

wherein X' is hydroxy; amino; or mono- or di-$C_{1-4}$alkyl amino; and W' is absent; -O-; -NH-; or $-CH_2-$;

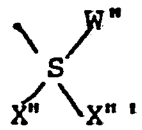

where X" and X"' are independently absent; or $\overset{O}{\overset{\uparrow}{S}}$; and

W" is absent; $-CH_2-$; or $-\overset{R^8}{\overset{|}{C}}H-$, where $R^8$ is hydrogen or $C_{1-3}$ alkyl;

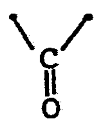 ; 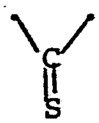 ; or

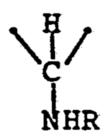 ; where R is hydrogen; $C_{1-4}$ alkyl; formyl; $C_{1-4}$ alkanoyl; aroyl; carboxy; $C_{1-4}$ alkoxycarbonyl; aryloxycarbonyl; or aryl $C_{1-4}$ alkoxycarbonyl; or

(2) 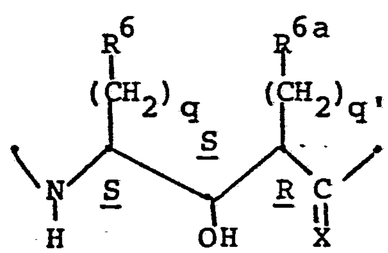 where q is 1 to 4; q' is 0 to 4; X is O or H, H;

$R^6$ is as defined above; and

$R^{6a}$ is hydrogen; $C_{1-8}$alkyl; $C_{2-8}$alkyl substituted with one or two members independently selected from the group consisting of hydroxy, carboxy, carboxy ester or amide, amino, mono-, di-, or tri-$C_{1-4}$alkylamino, and guanidyl; wherein said substitution occurs on the last 1 or 2 carbon atoms of the alkyl chain; aryl; $C_{3-7}$cycloalkyl; or aryl or $C_{3-7}$cycloalkyl substituted with up to three members selected from the group consisting of $C_{1-4}$alkyl, trifluoromethyl, hydroxy, $C_{1-4}$alkoxy, and halo; wherein the substituent of the above formula has 2<u>R</u>, 3<u>S</u>, 4<u>S</u> configuration;

J is (1) $-Y-(CH_2)_n-R^7$

where

Y is -NH- or -O-;

n is 0 to 5; and

$R^7$ is hydrogen, <u>provided</u> that where n is 0 and $R^7$ is hydrogen, that G is other than Sta and E is other than Phe; hydroxy; $C_{1-4}$alkyl; $C_{3-7}$cycloalkyl; aryl; aryl substituted with up to five members independently selected from the group consisting of $C_{1-6}$alkyl, trifluoromethyl, hydroxy, $C_{1-4}$alkoxy, amino, mono- or di- $C_{1-4}$ alkylamino, and halo; $N(R')_2$, where R' may be the same or different and is hydrogen, $C_{1-4}$alkyl, aryl, aryl $C_{1-4}$alkyl, heterocyclic, or heterocyclic $C_{1-4}$alkyl;

$N(R')^{\oplus}{}_3A^{\ominus}$, where R' is as defined above, and $A^{\ominus}$ is a counterion; guanidyl; heterocyclic; heterocyclic substituted with up to five members independently selected from the group consisting of $C_{1-6}$alkyl, hydroxy, trifluoromethyl, $C_{1-4}$alkoxy, halo, aryl, aryl $C_{1-4}$alkyl, amino, and mono- or di-$C_{1-4}$alkylamino; or heterocyclic substituted with another, the same or different, heterocyclic;

(2) 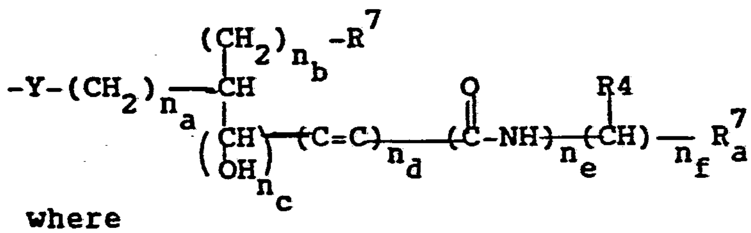

where

Y is as defined above;

$n_a$ is 0 or 1;

$n_b$ is 1 to 4;

$n_c$ is 0 or 1;

$n_d$ is 0 or 1;

$n_e$ is 0 or 1, provided that $n_e$ cannot be 1 when $n_d$ is 0;

$n_f$ is 1 to 4;

$R^4$ is hydrogen; or $-\underset{R^3}{CH}-R^9$, where

$R^9$ is hydrogen; $C_{1-4}$alkyl; hydroxy; or $C_{3-7}$cycloalkyl; and $R^3$ is hydrogen; $C_{1-4}$alkyl; aryl; aryl $C_{1-4}$alkyl;

aryl $C_{1-4}$alkyl or aryl substituted with up to three members selected from the group consisting of $C_{1-4}$alkyl, trifluoromethyl, hydroxy, $C_{1-4}$alkoxy, and halo; or indolyl; and

$R^7$ and $R^7_a$ may be the same or different and have the same meaning as $R^7$ above and $R^7_a$ may additionally be

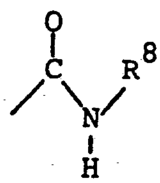 or 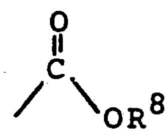,

where $R^8$ is hydrogen or $C_{1-3}$alkyl;

(3) 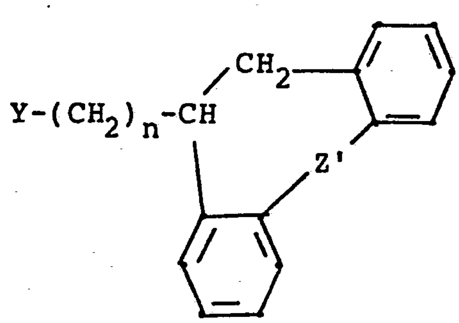

where

Y is as defined above;

n is 0 or 1; and

Z' is

(a) $-(CH_2)_n-\underset{R^8}{\underset{|}{CH}}-$

where

n is 0 or 1; and

$R^8$ is as defined above; or

(b) $-(CH_2)_n-\underset{\underset{CH_2}{\|}}{C}-$

where

n is 0 or 1; or

(4) (a) $Y-(\overset{R^{10}}{\overset{|}{C}H})_q-R^{11}$; (b) $Y-(\overset{R^{12}}{\overset{|}{C}H})_{q'}-R^{13}$; or

(c) $Y-\underset{\underset{R^{14}}{|}}{C}H-R^{11}$

where

Y is -NH- or -O-;

q is 1-5;

q' is 0-5;

$R^{10}$ is hydrogen; hydroxy; $N(R'')_2$, where R" may be the same or different and is hydrogen or $C_{1-4}$alkyl; guanidyl; or $N^{\oplus}(R'')_3A^{\ominus}$, where R" is as defined above, and $A^{\ominus}$ is a counterion; provided that at least one $R^{10}$ is not hydrogen;

$R^{11}$ is $C_{1-4}$alkyl; $C_{3-7}$cycloalkyl; aryl; aryl substituted with up to three members independently selected from the group consisting of $C_{1-6}$alkyl, trifluoromethyl, hydroxy, $C_{1-4}$alkoxy, amino, mono- or di- $C_{1-4}$alkylamino, amino

$C_{1-4}$alkyl, mono-, di-, or tri-$C_{1-4}$alkylamino-$C_{1-4}$alkyl, halo, carboxy, carboxy ester or amide, carboxy-$C_{1-4}$alkoxy, carboxy-$C_{1-4}$-alkoxy ester or amide, α-aminocarboxy-$C_{1-4}$alkyl, α-aminocarboxy-$C_{1-4}$alkyl ester or amide, carboxy-$C_{1-4}$alkyl, carboxy-$C_{1-4}$alkyl ester or amide, guanidyl, and guanidyl-$C_{1-4}$alkyl; carboxy, ester or amide; sulfo; heterocyclic; or heterocyclic substituted with up to five members independently selected from the group consisting of $C_{1-6}$alkyl, hydroxy, trifluoromethyl, $C_{1-4}$alkoxy, halo, aryl, aryl $C_{1-4}$alkyl, amino, and mono- or di-$C_{1-4}$alkylamino;

$R^{12}$ is hydrogen; or carboxy, ester or amide;

$R^{13}$ is carboxy, ester or amide; sulfo; or aryl substituted with up to three members selected from the group consisting of amino-$C_{1-4}$alkyl, mono-, di-, or tri-$C_{1-4}$-alkylamino-$C_{1-4}$-alkyl, halo, carboxy, carboxy ester or amide, carboxy-$C_{1-4}$alkoxy, carboxy-$C_{1-4}$alkoxy ester or amide,

α-amino-carboxy-$C_{1-4}$alkyl, α-aminocarboxy-$C_{1-4}$alkyl ester or amide, carboxy-$C_{1-4}$alkyl, carboxy-$C_{1-4}$alkyl ester or amide, guanidyl, and guanidyl-$C_{1-4}$alkyl; and

$R^{14}$ is carboxy, ester or amide;

(d) 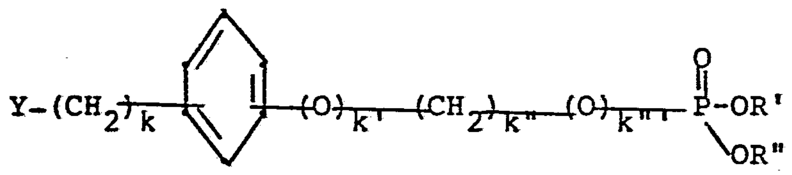 ; or

(e) 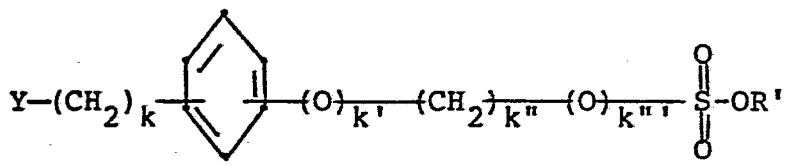 ,

where

Y is -NH- or -O-;

k is 0-4;

k' is 0 or 1;

k" is 0-4;

k"'is 0 or 1;

R' is hydrogen or $C_{1-4}$alkyl; and

R" is hydrogen or $C_{1-4}$alkyl;

$R^1$ is hydrogen; $C_{1-4}$ alkyl; hydroxy $C_{1-4}$alkyl; aryl; aryl substituted with up to three members selected from the group consisting of $C_{1-4}$ alkyl, trifluoromethyl, hydroxy, $C_{1-4}$ alkoxy, and halo; indolyl; 4-imidazolyl; amino $C_{2-4}$ alkyl; acyl $C_{2-4}$ alkyl wherein the acyl is $R^9$-C(=O)- and $R^9$ is as defined above; guanidyl $C_{2-3}$ alkyl; or methylthiomethyl;

wherein all of the asymmetric carbon atoms have an S configuration, except for those in the B, D, and G substituents, which may have an S or R configuration;

and a pharmaceutically acceptable salt thereof.

2. A peptide according to Claim 1 wherein the peptide is a member selected from the group consisting essentially of:

BOC-His-Pro-Phe-His-Sta-OEt

BOC-Phe-His- 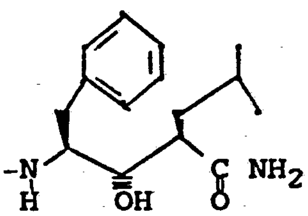

BOC-Phe-His- 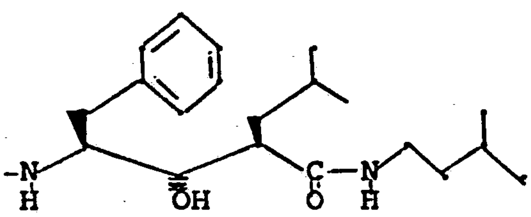

BOC-Phe-His-ACHPA-$NH_2$

BOC-HomoPhe-His-Sta-$NH_2$

BOC-Phe-His- 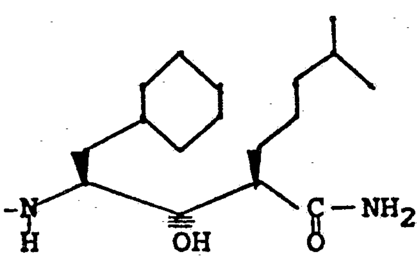

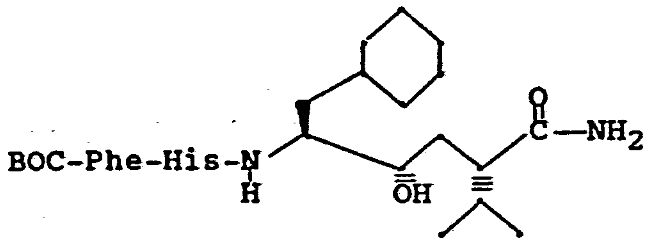

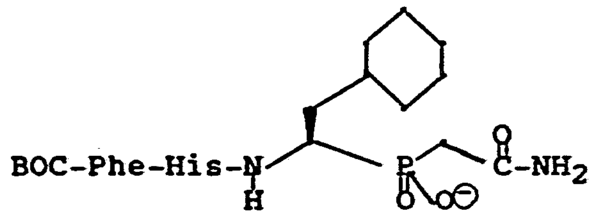

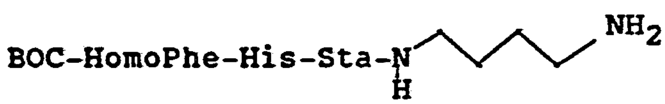

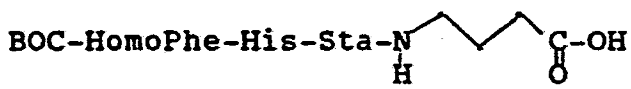

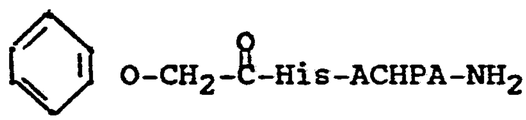

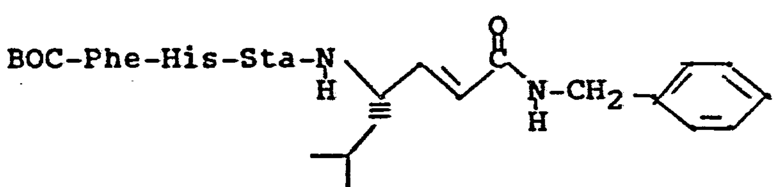

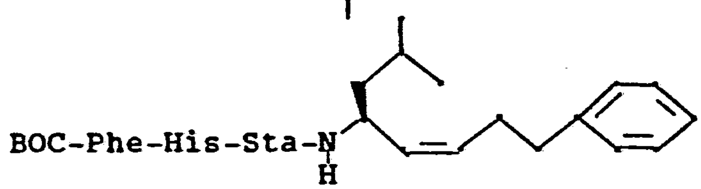

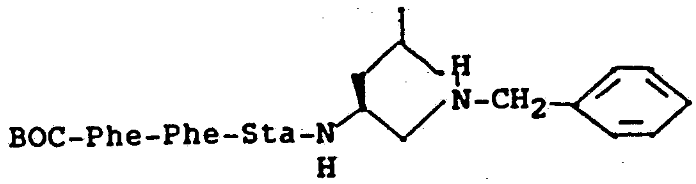

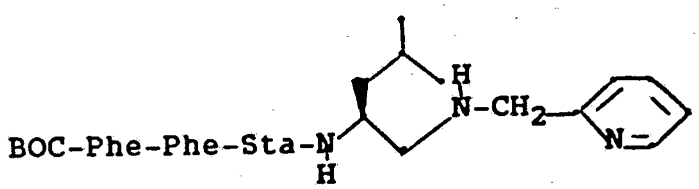

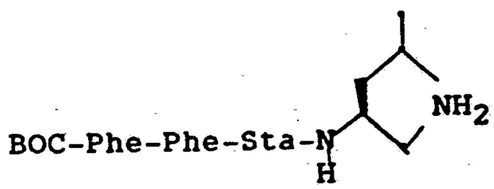

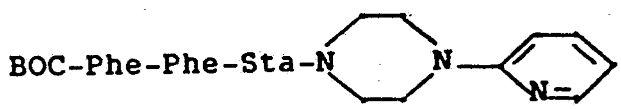

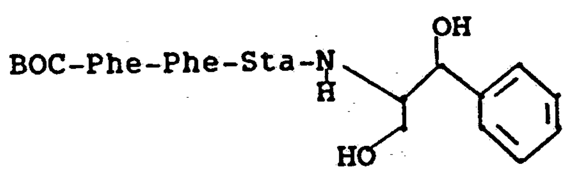

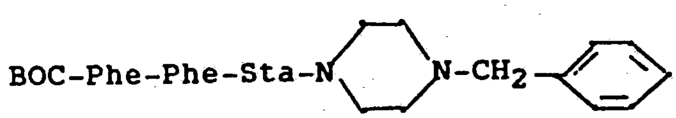

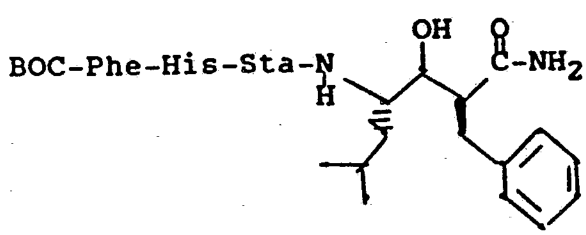

BOC-Phe-Phe-Sta- 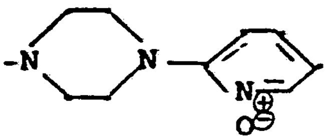

BOC-Phe-His-AHPPA-NH 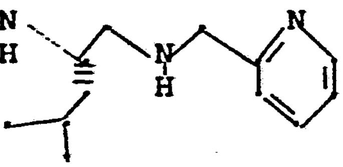

3. A pharmaceutical composition for treating renin-associated hypertension, comprising a pharmaceutical carrier and a therapeutically effective amount of a peptide according to Claim 1.

4. A pharmaceutical composition for treating renin-associated hypertension, comprising a pharmaceutical carrier and a therapeutically effective amount of a peptide according to Claim 2.

5. A method of treating renin-associated hypertension comprising administering to a patient in need of such treatment, a therapeutically effective amount of a peptide according to Claim 1.

6. A pharmaceutical composition for treating renin-associated hyperaldosteronism, comprising a pharmaceutical carrier and a therapeutically effective amount of a peptide according to Claim 1.

7. A combination for treating hypertension comprising a peptide of the formula:

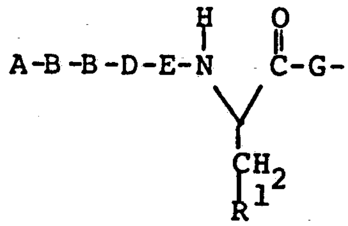

(I.)

wherein:

A is hydrogen; or 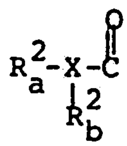

where

X is -O-; -O-CH-; -CH-O-; -CH-; -NH-CH-; or -S-CH-; and

$R_a^2$ and $R_b^2$ may be the same or different and are hydrogen; W-$(CH_2)_n$- or W-$(CH_2)_m$-CH=CH-$(CH_2)_p$, where W is hydrogen; $C_{1-4}$alkyl; aryl;

$C_{3-7}$cycloalkyl; or $C_{3-7}$cycloalkyl or aryl substituted with up to five members independently selected from the group consisting of $C_{1-8}$alkyl, trifluoromethyl, hydroxy, $C_{1-4}$alkoxy, and halo; n is 0 to 5; m is 0 to 2; and p is 0 to 2; except that where X is -O-, only one of $R^2_a$ or $R^2_b$ is present;

B is absent; glycyl; sarcosyl; or 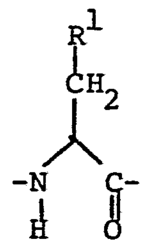 ,

where $R^1$ is as defined further below;

D is absent; or 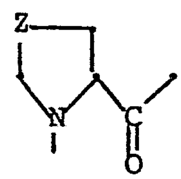 , where Z is

$-(CH_2)_l-$ and l is 1 or 2; or -S-;

E is absent; or 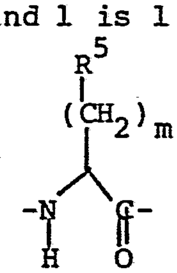 , where m is 1 to 4; and

$R^5$ is hydrogen; $C_{1-4}$ alkyl; aryl; aryl-$C_{1-4}$ alkyl; aryl $C_{1-4}$ alkyl or aryl where the aryl portion is substituted with up to three members selected from the group consisting of $C_{1-4}$ alkyl, trifluoromethyl, hydroxy, $C_{1-4}$ alkoxy, and halo; or indolyl;

G is (1) 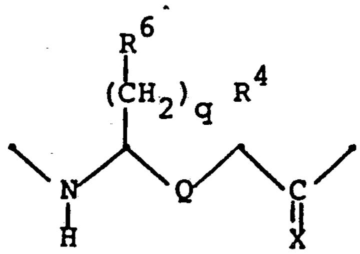 where q is 1 to 4; X is O, or H, H;

$R^4$ is hydrogen; or 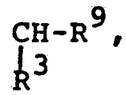,

where $R^9$ is hydrogen; $C_{1-4}$alkyl; hydroxy, or $C_{3-7}$cycloalkyl; and $R^3$ is hydrogen; $C_{1-4}$alkyl; aryl; aryl $C_{1-4}$alkyl; aryl $C_{1-4}$alkyl or aryl substituted with up to three members selected from the group consisting of $C_{1-4}$alkyl, trifluoromethyl, hydroxy, $C_{1-4}$alkoxy, and halo; or indolyl;

$R^6$ is $C_{3-6}$ alkyl; $C_{3-7}$ cycloalkyl; aryl; or $C_{3-7}$cycloalkyl or aryl substituted with up to three members selected from the group consisting of $C_{1-4}$alkyl, trifluoromethyl, hydroxy, $C_{1-4}$alkoxy, and halo; and

Q is 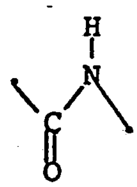 ; 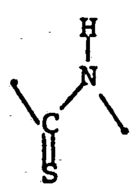 ; 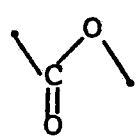 ; 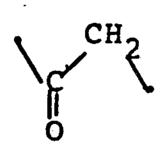 ;

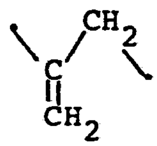 ; 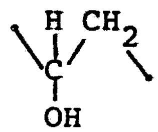 ; 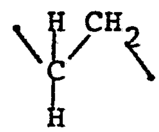 ; 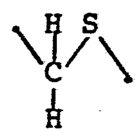 ;

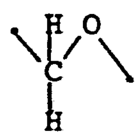 ; 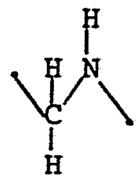 ; 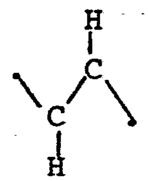 ; 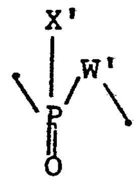 ;

wherein X' is hydroxy; amino; or mono- or di-$C_{1-4}$alkyl amino; and W' is absent; -O-; -NH-; or $-CH_2-$;

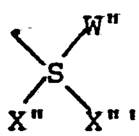

where X" and X"' are independently absent; or $\overset{O}{\uparrow}S$; and W" is absent; $-CH_2-$; or $-\overset{R^8}{|}CH-$, where $R^8$ is hydrogen or $C_{1-3}$ alkyl;

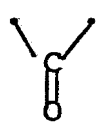 ; 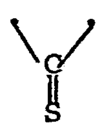 ; or

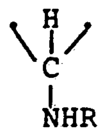 ; where R is hydrogen; $C_{1-4}$ alkyl; formyl; $C_{1-4}$ alkanoyl; aroyl; carboxy; $C_{1-4}$ alkoxycarbonyl; aryloxycarbonyl; or aryl $C_{1-4}$ alkoxycarbonyl; or

(2) 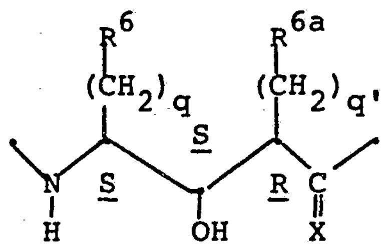 where q is 1 to 4; q' is 0 to 4; X is O or H, H;

$R^6$ is as defined above; and

$R^{6a}$ is hydrogen; $C_{1-8}$alkyl; $C_{2-8}$alkyl substituted with one or two members independently selected from the group consisting of hydroxy, carboxy, carboxy ester or amide, amino, mono-, di-, or tri-$C_{1-4}$alkylamino, and guanidyl; wherein said substitution occurs on the last 1 or 2 carbon atoms of the alkyl chain; aryl; $C_{3-7}$cycloalkyl; or aryl or $C_{3-7}$cycloalkyl substituted with up to three members selected from the group consisting of $C_{1-4}$alkyl, trifluoromethyl, hydroxy, $C_{1-4}$alkoxy, and halo;

wherein the substituent of the above formula has 2<u>R</u>, 3<u>S</u>, 4<u>S</u> configuration;

J is (1) $-Y-(CH_2)_n-R^7$

where

Y is -NH- or -O-;

n is 0 to 5; and

$R^7$ is hydrogen, <u>provided</u> that where n is 0 and $R^7$ is hydrogen, that G is other than Sta and E is other than Phe; hydroxy; $C_{1-4}$alkyl; $C_{3-7}$cycloalkyl; aryl; aryl substituted with up to five members independently selected from the group consisting of $C_{1-6}$alkyl, trifluoromethyl, hydroxy, $C_{1-4}$alkoxy, amino, mono- or di- $C_{1-4}$ alkylamino, and halo; $N(R')_2$, where R' may be the same or different and is hydrogen, $C_{1-4}$alkyl, aryl, aryl $C_{1-4}$alkyl, heterocyclic, or heterocyclic $C_{1-4}$alkyl; $N(R')^{\oplus}_3A^{\ominus}$, where R' is as defined above, and $A^{\ominus}$ is a counterion; guanidyl; heterocyclic; heterocyclic substituted with up to five members independently selected from the group consisting of $C_{1-6}$alkyl, hydroxy, trifluoromethyl, $C_{1-4}$alkoxy, halo, aryl, aryl $C_{1-4}$alkyl, amino, and mono- or di-$C_{1-4}$alkylamino; or heterocyclic substituted with another, the same or different, heterocyclic;

(2) 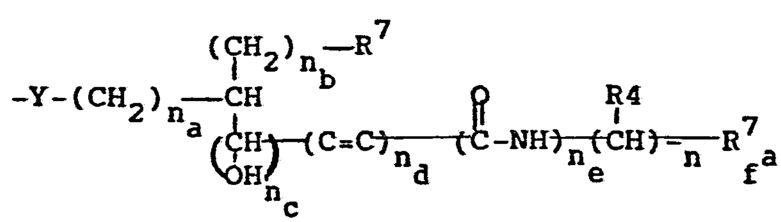

where

Y is as defined above;

$n_a$ is 0 or 1;

$n_b$ is 1 to 4;

$n_c$ is 0 or 1;

$n_d$ is 0 or 1;

$n_e$ is 0 or 1, provided that $n_e$ cannot be 1 when $n_d$ is 0;

$n_f$ is 1 to 4;

$R^4$ is hydrogen; or $-\underset{R^3}{\underset{|}{CH}}-R^9$, where

$R^9$ is hydrogen; $C_{1-4}$alkyl; hydroxy; or $C_{3-7}$cycloalkyl; and $R^3$ is hydrogen; $C_{1-4}$alkyl; aryl; aryl $C_{1-4}$alkyl; aryl $C_{1-4}$alkyl or aryl substituted with up to three members selected from the group consisting of $C_{1-4}$alkyl, trifluoromethyl, hydroxy, $C_{1-4}$alkoxy, and halo; or indolyl; and

$R^7$ and $R^7_a$ may be the same or different and have the same meaning as $R^7$ above and $R^7_a$ may additionally be

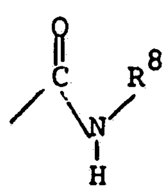 or 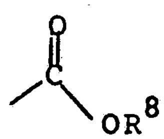,

where $R^8$ is hydrogen or $C_{1-3}$alkyl;

(3) 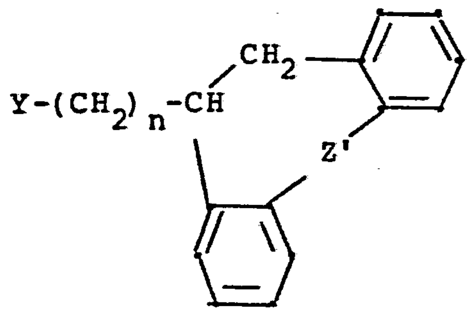

where

Y is as defined above;

n is 0 or 1; and

Z' is

(a) $-(CH_2)_n-\underset{R^8}{\underset{|}{CH}}-$

where

n is 0 or 1; and

$R^8$ is as defined above; or

(b) $-(CH_2)_n-\underset{CH_2}{\underset{||}{C}}-$

where

n is 0 or 1; or

(4) (a) $Y-(\overset{R^{10}}{\overset{|}{CH}})_q-R^{11}$; (b) $Y-(\overset{R^{12}}{\overset{|}{CH}})_{q'}-R^{13}$; or

(c) $Y-\underset{R^{14}}{\underset{|}{CH}}-R^{11}$

where

Y is -NH- or -O-;

q is 1-5;

q' is 0-5;

$R^{10}$ is hydrogen; hydroxy; $N(R'')_2$, where R" may be the same or different and is hydrogen or $C_{1-4}$alkyl; guanidyl; or $N^{\oplus}(R'')_3A^{\ominus}$, where R" is as defined above, and $A^{\ominus}$ is a counterion; provided that at least one $R^{10}$ is not hydrogen;

$R^{11}$ is $C_{1-4}$alkyl; $C_{3-7}$cycloalkyl; aryl; aryl substituted with up to three members independently selected from the group consisting of $C_{1-6}$alkyl, trifluoromethyl, hydroxy, $C_{1-4}$alkoxy, amino, mono- or di- $C_{1-4}$alkylamino, amino $C_{1-4}$alkyl, mono-, di-, or tri-$C_{1-4}$alkylamino-$C_{1-4}$alkyl, halo, carboxy, carboxy ester or amide, carboxy-$C_{1-4}$alkoxy, carboxy-$C_{1-4}$-alkoxy ester or amide, α-aminocarboxy-$C_{1-4}$alkyl, α-aminocarboxy-$C_{1-4}$alkyl ester or amide, carboxy-$C_{1-4}$alkyl, carboxy-$C_{1-4}$alkyl ester or amide, guanidyl, and guanidyl-$C_{1-4}$alkyl; carboxy, ester or amide; sulfo; heterocyclic; or heterocyclic substituted with up to five members independently selected from the group consisting of $C_{1-6}$alkyl,

hydroxy, trifluoromethyl, C alkoxy, halo, aryl$_{1-4}$aryl $C_{1-4}$alkyl, amino, and mono- or di-$C_{1-4}$alkylamino;

$R^{12}$ is hydrogen; or carboxy, ester or amide;

$R^{13}$ is carboxy, ester or amide; sulfo; or aryl substituted with up to three members selected from the group consisting of amino-$C_{1-4}$alkyl, mono-, di-, or tri-$C_{1-4}$-alkylamino-$C_{1-4}$-alkyl, halo, carboxy, carboxy ester or amide, carboxy-$C_{1-4}$alkoxy, carboxy-$C_{1-4}$alkoxy ester or amide, α-amino-carboxy-$C_{1-4}$alkyl, α-aminocarboxy-$C_{1-4}$alkyl ester or amide, carboxy-$C_{1-4}$alkyl, carboxy-$C_{1-4}$alkyl ester or amide, guanidyl, and guanidyl-$C_{1-4}$alkyl; and

$R^{14}$ is carboxy, ester or amide;

(d) 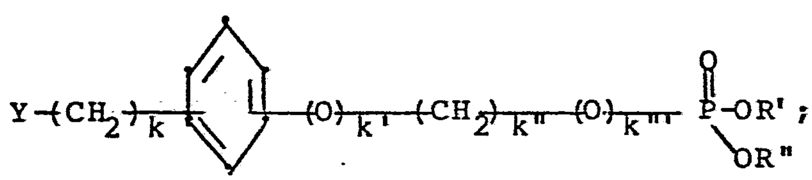 or

(e) 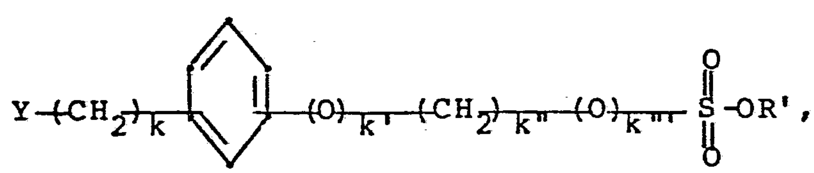

where
Y is -NH- or -O-;
k is 0-4;
k' is 0 or 1;
k" is 0-4;
k"'is 0 or 1;
R' is hydrogen or $C_{1-4}$alkyl; and
R" is hydrogen or $C_{1-4}$alkyl;

$R^1$ is hydrogen; $C_{1-4}$ alkyl; hydroxy $C_{1-4}$alkyl; aryl; aryl substituted with up to three members selected from the group consisting of $C_{1-4}$ alkyl, trifluoromethyl, hydroxy, $C_{1-4}$ alkoxy, and halo; indolyl; 4-imidazolyl; amino $C_{2-4}$ alkyl; acyl $C_{2-4}$ alkyl wherein the acyl is $R^9-\overset{O}{\overset{\|}{C}}-$ and $R^9$ is as defined above; guanidyl $C_{2-3}$ alkyl; or methylthiomethyl;

wherein all of the asymmetric carbon atoms have an S configuration, except for those in the B, D, and G substituents, which may have an S or R configuration; and a pharmaceutically acceptable salt thereof; and one or more antihypertensive agents selected from the group consisting essentially of:

Diuretics: acetazolamide; amiloride; bendroflumethiazide; benzthiazide; bumetanide; chlorothiazide; chlorthalidone; cyclothiazide; ethacrynic acid; furosemide; hydrochlorothiazide; hydroflumethiazide; indacrinone (racemic mixture, or as either the (+) or (-) enantiomer alone, or a

manipulated ratio, e.g., 9:1 of said enantiomers, respectively); metolazone; methyclothiazide; muzolimine; polythiazide; quinethazone; sodium ethacrynate; sodium nitroprusside; spironolactone; ticrynafen; triamterene; trichlormethiazide;

<u>α-Adrenergic Blocking Agents:</u> dibenamine; phentolamine; phenoxybenzamine; prazosin; tolazoline;

<u>ß-Adrenergic Blocking Agents:</u> atenolol; metoprolol; nadolol; propranolol; timolol;
((±)-2-[3-(tert-butylamino)-2-hydroxypropoxy]-2-furananilide) (ancarolol);
(2-acetyl-7-(2-hydroxy-3-isopropylaminopropoxy)benzofuran HCl) (befunolol);
((±)-1-(isopropylamino)-3-(p-(2-cyclopropylmethoxyethyl)-phenoxy)-2-propranol HCl) (betaxolol);
(1-[(3,4-dimethoxyphenethyl)amino]-3-(m-tolyloxy)-2-propanol HCl) (bevantolol);
(((±)-1-(4-((2-isopropoxyethoxy)methyl)phenoxy)-3-isopropylamino-2-propanol)fumarate) (bisoprolol);
(4-(2-hydroxy-3-[4-(phenoxymethyl)-piperidino]-propoxy)-indole);
(carbazolyl-4-oxy-5,2-(2-methoxyphenoxy)-ethylamino-2-propanol);
(1-((1,1-dimethylethyl)amino)-3-((2-methyl-1H-indol-4-yl)oxy)-2-propanol benzoate) (bopindolol);
(1-(2-exobicyclo[2.2.1]-hept-2-ylphenoxy)-3-[(1-methylethyl)-amino]-2-propanol HCl) (bornaprolol);
(o-[2-hydroxy-3-[(2-indol-3-yl-1,1-dimethylethyl)-amino]propoxy]benzonitrile HCl) (bucindolol);

(α- [(tert.butylamino)methyl]- 7-ethyl- 2-benzofuranmethanol) (bufuralol);
(3- [3-acetyl- 4- [3-(tert.butylamino)-2-hydroxypropyl]-phenyl]-1,1-diethylurea HCl) (celiprolol);
((±)-2- [2- [3- [(1,1-dimethylethyl)amino]-2-hydroxypropoxy]phenoxy]-N-methylacetamide HCl) (cetamolol);
(2-benzimidazolyl-phenyl(2-isopropylaminopropanol));
((±)-3'-acetyl-4'-(2-hydroxy-3-isopropylaminopropoxy)-acetanilide HCl) (diacetolol);
(methyl-4-[2-hydroxy-3-[(1-methylethyl)aminopropoxy]]-benzenepropanoate HCl) (esmolol);
(erythro-DL-1-(7-methylindan-4-yloxy)-3-isopropylaminobutan-2-ol);
(1-(tert.butylamino)-3-[O-(2-propynyloxy)phenoxy]-2-propanol (pargolol);
(1-(tert.butylamino)-3-[o-(6-hydrazino-3-pyridazinyl)-phenoxy]-2-propanol diHCl) (prizidilol);
((-)-2-hydroxy-5-[(R)-1-hydroxy-2-[(R)-(1-methyl-3-phenylpropyl)amino]ethyl]benzamide);
(4-hydroxy-9-[2-hydroxy-3-(isopropylamino)-propoxy]-7-methyl-5H-furo[3,2-g][1]-benzopyran-5-one) (iprocrolol);
((-)-5-(tert.butylamino)-2-hydroxypropoxy]-3,4-dihydro-1-(2H)-naphthalenone HCl) (levobunolol);
(4-(2-hydroxy-3-isopropylamino-propoxy)-1,2-benzisothiazole HCl);
(4-[3-(tert.butylamino)-2-hydroxypropoxy]-N-methylisocarbostyril HCl);
((±)-N-2-[4-(2-hydroxy-3-isopropyl aminopropoxy)-phenyl]ethyl-N'-isopropylurea) (pafenolol);

(3- [[(2-trifluoroacetamido)ethyl] amino]-1-phenoxy-propan-2-ol);

(N-(3-(o-chlorophenoxy)-2-hydroxypropyl)-N'-(4'-chloro-2,3-dihydro-3-oxo-5-pyridazinyl)ethylenediamine);

((±)-N-[3-acetyl-4-[2-hydroxy-3-[(1-methylethyl)amino]-propoxy] phenyl] butanamide) (acebutolol);

((±)-4'-[3-(tert-butylamino)-2-hydroxypropoxy] spiro-[cyclohexane-1,2'-indan]-1'-one) (spirendolol);

(7-[3-[[2-hydroxy-3-[(2-methylindol-4-yl)oxy] propyl]-amino] butyl] thiophylline) (teoprolol);

((±)-1-tert.butylamino-3-(thiochroman-8-yloxy)-2-propanol) (tertatolol);

((±)-1-tert.butylamino-3-(2,3-xylyloxy)-2-propanol HCl) (xibenolol);

(8-[3-(tert.butylamino)-2-hydroxypropoxy]-5-methyl-coumarin) (bucumolol);

(2-(3-(tert.butylamino)-2-hydroxy-propoxy)benzonitrile HCl) (bunitrolol);

((±)-2'-[3-(tert-butylamino)-2-hydroxypropoxy-5'-fluorobutyrophenone) (butofilolol);

(1-(carbazol-4-yloxy)-3-(isopropylamino)-2-propanol) (carazolol);

(5-(3-tert.butylamino-2-hydroxy)propoxy-3,4-dihydro-carbostyril HCl) (carteolol);

(1-(tert.butylamino)-3-(2,5-dichlorophenoxy)-2-propanol) (cloranolol);

(1-(inden-4(or 7)-yloxy)-3-(isopropylamino)-2-propanol HCl) (indenolol);

(1-isopropylamino-3-[(2-methylindol-4-yl)oxy]-2-propanol) (mepindolol);

(1-(4-acetoxy-2,3,5-trimethylphenoxy)-3-isopropylaminopropan-2-ol) (metipranolol);

(1-(isopropylamino)-3-(o-methoxyphenoxy)-3-[(1-methylethyl)amino]-2-propanol) (moprolol);

((1-tert.butylamino)-3-[(5,6,7,8-tetrahydro-cis-6,7-dihydroxy-1-naphthyl)oxy]-2-propanol) (nadolol);

((S)-1-(2-cyclopentylphenoxy)-3-[(1,1-dimethylethyl)-amino]-2-propanol sulfate (2:1)) (penbutolol);

(4'-[1-hydroxy-2-(amino)ethyl]methanesulfonanilide) (sotalol);

(2-methyl-3-[4-(2-hydroxy-3-tert.butylaminopropoxy)-phenyl]-7-methoxy-isoquinolin-1-(2H)-one);

(1-(4-(2-(4-fluorophenyloxy)ethoxy)phenoxy)-3-isopropylamino-2-propanol HCl);

((-)-p-[3-[(3,4-dimethoxyphenethyl)amino]-2-hydroxy-propoxy]-ß-methylcinnamonitrile) (pacrinolol);

((±)-2-(3'-tert.butylamino-2'-hydroxypropylthio)-4-(5'-carbamoyl-2'-thienyl)thiazole HCl) (arotinolol);

((±)-1-[p-[2-(cyclopropylmethoxy)ethoxy]phenoxy]-3-(isopropylamino)-2-propanol) (cicloprolol);

((±)-1-[(3-chloro-2-methylindol-4-yl)oxy]-3-[(2-phenoxyethyl)amino]-2-propanol) (indopanolol);

((±)-6-[[2-[[3-(p-butoxyphenoxy)-2-hydroxypropyl]-amino]ethyl]amino]-1,3-dimethyluracil) (pirepolol);

(4-(cyclohexylamino)-1-(1-naphtholenyloxy)-2-butanol);

(1-phenyl-3-[2-[3-(2-cyanophenoxy)-2-hydroxypropyl]-aminoethyl]hydantoin HCl);

(3,4-dihydro-8-(2-hydroxy-3-isopropylaminopropoxy)-3-nitroxy-2H-1-benzopyran) (nipradolol);

<u>α and ß-Adrenergic Blocking Agents</u>:
((±)-1-tert-butylamino)-3-[o-[2-(3-methyl-5-isoxazolyl)vinyl]phenoxy]-2-propanol) (isoxaprolol);
(1-isopropylamino-3-(4-(2-nitroxyethoxy)phenoxy)-2-propanol HCl);
(4-hydroxy-α-[[3-(4-methoxyphenyl)-1-methylpropyl]-aminomethyl]-3-(methylsulfinyl)-benzmethanol HCl) (sulfinalol);
(5-[1-hydroxy-2-[[2-(o-methoxyphenoxy)ethyl]amino]-ethyl]-2-methylbenzenesulfonamide HCl);
(5-[1-hydroxy-2-[(1-methyl-3-phenylpropyl)amino]ethyl]-salicylamide HCl) (labetalol);
(1-((3-chloro-2-methyl-1H-indol-4-yl)oxy)-3-((2-phenoxyethyl)amino)-2-propanol-hydrogenmalonate) (ifendolol);
(4-(2-hydroxy-3-[(1-methyl-3-phenylpropyl)amino]-propoxy)benzeneacetamide);
(1-[3-[[3-(1-naphthoxy)-2-hydroxypropyl]-amino]-3,3-dimethyl-propyl]-2-benzimidazolinone);
(3-(1-(2-hydroxy-2-(4-chlorophenylethyl)-4-piperidyl)-3,4-dihydroxy)quinoxolin-2(1H)-one);

<u>CNS-Acting Agents</u>: clonidine; methyldopa;

<u>Adrenergic Neuron Blocking Agents</u>: guanethidine; reserpine and other rauwolfia alkaloids such as rescinnamine;

<u>Vasodilators</u>: diazoxide; hydralazine; minoxidil;

<u>Calcium Channel Blockers</u>:

α-[3-[[2-(3,4-dimethoxyphenyl)ethyl]methylamino]-propyl]-3,4-dimethoxy-α-(1-methylethyl)benzene-acetonitrile (verapamil);

1,4-dihydro-2,6-dimethyl-4-(2-nitrophenyl)-3,5-pyridinedicarboxylic acid dimethyl ester (nifedipine);

2-(2,2-dicyclohexylethyl)piperidine (perhexiline);

N-(1-methyl-2-phenylethyl)- -phenylbenzenepropanamine (prenylamine);

3-(aminosulfonyl)-4-chloro-N-(2,3-dihydro-2-methyl-1H-indol-1-yl)benzamide (indapamide);

(2'-(2-diethylaminoethoxy)-3-phenylpropiophenone (etafenone);

(4-[4,4-bis-(4-fluorophenyl)butyl]-N-(2,6-dimethylphenyl)-1-piperazineacetamide) (lidoflazine);

(2-(N-benzyl-N-methylamino)ethylmethyl-2,6-dimethyl-4-(m-nitrophenyl)-1,4-dihydro-3,5-pyridinedicar-boxylate HCl) (nicardipine);

(N-(3,4-dimethoxyphenethyl)-2-(3,4-dimethoxyphenyl)-N-methyl-m-dithiane-2-propylamine-1,1,3,3-tetraoxide) (tiapamil);

(5,6-dimethoxy-2-(3-[(α-(3,4-dimethoxy)phenylethyl)-methylamino]propyl)phthalimidine) (falipamil);

(ß-[(2-methylpropoxy)methyl]-N-phenyl-N-phenylmethyl-1-pyrrolidineethanamine HCl monohydrate) (bepridil);

((+)-cis-3-(acetyloxy)-5-[2-(dimethylamino)ethyl]-2,3-dihydro-2-(4-methoxyphe yl)-1,5-benzothiazepin-4-(5H)-one) (diltiazem);

((E)-1-[bis-(p-fluorophenyl)methyl]-4-cinnamylpiperazine di HCl) (flunarizine);

(5-[(3,4-dimethoxyphenethyl)methylamino]-2-isopropyl-2-(3,4,5-trimethoxyphenyl)valeronitrile (gallopamil);

(ethylmethyl(2,3-dichlorophenyl)-1,4-dihydro-2,6-dimethyl-3,5-pyridinedicarboxylate (felodipine);

(isopropyl-2-methoxyethyl-1,4-dihydro-2,6-dimethyl-4-(3-nitrophenyl)-3,5-pyridinecarboxylate) (nimodipine);

(3-ethyl-5-methyl-1,4-dihydro-2,6-dimethyl-4-(3-nitrophenyl)-3,5-pyridine-dicarboxylate) (nitrendipine);

<u>Angiotensin I Converting Enzyme Inhibitors</u>:

1-(3-mercapto-2-methyl-1-oxopropyl)-L-proline (captopril);

(1-(4-ethoxycarbonyl-2,4(R,R)-dimethylbutanoyl)-indoline-2(S)-carboxylic acid);

(2-[2-[[1-(ethoxycarbonyl)-3-phenyl-propyl]amino]-1-oxopropyl]-1,2,3,4-tetrahydro-3-isoquinoline carboxylic acid);

((S)-1-[2-[[1-(ethoxycarbonyl)-3-phenylpropyl]amino]-1-oxopropyl]octahydro-1H-indole-2-carboxylic acid HCl);

(N-cyclopentyl-N-(3-(2,2-dimethyl-1-oxopropyl)thiol-2-methyl-1-oxopropyl)glycine) (pivalopril);

((2R,4R)-2-(2-hydroxyphenyl)-3-(3-mercaptopropionyl)-4-thiazolidinecarboxylic acid);

(1-(N-[1(S)-ethoxycarbonyl-3-phenylpropyl]-(S)-alanyl)-cis,syn-octahydroindol-2(S)-carboxylic acid HCl);

((-)-(S)-1-[(S)-3-mercapto-2-methyl-1-oxopropyl]-indoline-2-carboxylic acid);

([1(S),4S]-1-[3-(benzoylthio)-2-methyl-1-oxopropyl]-4-phenylthio-L-proline;

(3-([1-ethoxycarbonyl-3-phenyl-(1S)-propyl]amino)-2,3,4,5-tetrahydro-2-oxo-1-(3S)-benzazepine-1-acetic acid HCl);

(N-(2-benzyl-3-mercaptopropanoyl)-S-ethyl-L-cysteine) and the S-methyl analogue;

(N-(1(S)-ethoxycarbonyl-3-phenylpropyl)-L-alanyl-L-proline maleate) (enalapril);

N-[1-(S)-carboxy-3-phenylpropyl]-L-alanyl-1-proline;

$N^2$-[1-(S)-carboxy-3-phenylpropyl]-L-lysyl-L-proline (lysinopril);

<u>Other Antihypertensive Agents</u>: aminophylline; cryptenamine acetates and tannates; deserpidine; meremethoxylline procaine; pargyline; trimethaphan camsylate;

including pharmaceutical salt and ester forms thereof.